(19) Europäisches Patentamt
European Patent Office
Office européen des brevets

(11) Publication number: **0 219 814 B1**

(12) # EUROPEAN PATENT SPECIFICATION

(45) Date of publication of patent specification: **11.09.91**

(51) Int. Cl.[5]: **C12N 15/16**, //(C12N15/62, 15:70)

(21) Application number: **86114321.2**

(22) Date of filing: **16.10.86**

The file contains technical information submitted after the application was filed and not included in this specification

(54) **Process for production of isulin-like growth factor I and plasmid for production thereof.**

(30) Priority: **21.10.85 GB 8525929**
**14.07.86 GB 8617073**

(43) Date of publication of application:
**29.04.87 Bulletin 87/18**

(45) Publication of the grant of the patent:
**11.09.91 Bulletin 91/37**

(84) Designated Contracting States:
**AT BE CH DE ES FR GB GR IT LI LU NL SE**

(56) References cited:
**EP-A- 0 123 228**
**EP-A- 0 128 733**
**EP-A- 0 130 166**
**EP-A- 0 135 094**
**EP-A- 0 155 655**

(73) Proprietor: **FUJISAWA PHARMACEUTICAL CO., LTD.**
**3, Doshomachi 4-chome Higashi-ku**
**Osaka-shi Osaka 541(JP)**

(72) Inventor: **Ueda, Ikuo**
**No. 2-11-95 Uenohigashi**
**Toyonaka-shi Osaka-fu, 560(JP)**
Inventor: **Niwa, Mineo**
**No. 7-15 Kirinokuchi Kamiuenocho**
**Mukoo-shi Kyoto-fu, 617(JP)**
Inventor: **Satoh, Susumu**
**No. 7-9-4 Higashitokiwadai Toyono-cho**
**Toyono-gun Osaka-fu, 563-01(JP)**
Inventor: **Saitoh, Yoshimasa**
**No. 5-14-10 Higashitokiwadai Toyono-cho**
**Toyono-gun Osaka-fu, 563-01(JP)**
Inventor: **Kusunoki, Chihiro**
**B-407, No. 6, Satsukigaoka-higashi**
**Suita-shi Osaka-fu, 565(JP)**

(74) Representative: **Türk, Gille, Hrabal et al**
**Brucknerstrasse 20**
**W-4000 Düsseldorf 13(DE)**

EP 0 219 814 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an improved process for production of human insulin-like growth factor (hereinafter referred to as IGF-I).

More particularly, this invention relates to an improved process for production of IGF-I fused with a protective peptide (hereinafter referred to as fused IGF-I), which may be led to IGF-I by elimination reaction of protective peptide, to a corresponding expression plasmid and to a corresponding transformant for the production thereof.

IGF-I is known to consist of 70 amino acids as shown in the following sequence:

```
    1                                            10
    Gly-Pro-Glu-Thr-Leu-Cys-Gly-Ala-Glu-Leu-Val-Asp-
                                        20
Ala-Leu-Gln-Phe-Val-Cys-Gly-Asp-Arg-Gly-Phe-Tyr-Phe-
                 30
Asn-Lys-Pro-Thr-Gly-Tyr-Gly-Ser-Ser-Ser-Arg-Arg-Ala-
    40                                          50
Pro-Gln-Thr-Gly-Ile-Val-Asp-Glu-Cys-Cys-Phe-Arg-Ser-
                                        60
Cys-Asp-Leu-Arg-Arg-Leu-Glu-Met-Tyr-Cys-Ala-Pro-Leu-
                         70
Lys-Pro-Ala-Lys-Ser-Ala  .
```

The inventors of this invention succeeded in producing IGF-I in a good yield by using the following essential steps.

Step 1

A process for the production of a gene coding for fused IGF-I (hereinafter referred to as fused IGF-I gene).

Step 2

A process for the production of an expression plasmid which comprises inserting said fused IGF-I gene into a Co1E1 plasmid from which a rop gene is cut off.

Suitable "Co1E1 plasmid" may include pBR322 and the like.

Step 3

A process for the production of a transformant which comprises transforming a host organism with said expression plasmid.

Step 4

A process for production of fused IGF-I which comprises culturing said transformant in a suitable medium.

Step 5

A process for isolation of fused IGF-I from host organism cells.

Step 6

A process for the production of IGF-I which comprises subjecting said fused IGF-I to elimination reaction of the protective peptide.

The protective peptide is used for protecting IGF-I from degradation by protease in the cells of a host organism, and is removed by elimination reaction of said fused IGF-I.

Namely, said fused IGF-I is an intermediate for preparing IGF-I by elimination reaction, so said protective peptide can be any eliminable protective peptide derived from natural or synthetic protein, natural or synthetic peptide, or a fragment thereof.

Suitable "fused IGF-I" may include IGF-I fused with a protein peptide through methionine of the protein peptide.

One of the suitable example of protein peptide is "protein peptide LH", amino acid sequence of which is as follows.

```
1                                                  10
Cys-Tyr-Cys-Gln-Asp-Pro-Tyr-Val-Lys-Glu-Ala-Glu-Asn-Leu-

                            20
Lys-Lys-Tyr-Phe-Asn-Ala-Gly-His-Ser-Asp-Val-Ala-Asp-Asn-

    30                                             40
Gly-Thr-Leu-Phe-Leu-Gly-Ile-Leu-Lys-Asn-Trp-Lys-Glu-Glu-

                                    50
Ser-Asp-Arg-Lys-Ile-Met-Gln-Ser-Gln-Ile-Val-Ser-Phe-Tyr-

                60                      66
Phe-Lys-Leu-Glu-Val-Lys-His-Glu-Phe-Met.
```

Suitable gene coding for IGF-I fused with protein peptide LH (422 bp) is as follows.

```
                       (EcoRI)      1
                                 Met Cys Tyr Cys Gln Asp Pro Tyr
      Coding:     5'-AATTC-ATG-TGT-TAC-TGC-CAG-GAC-CCA-TAT-
      Noncoding:      3'-G-TAC-ACA-ATG-ACG-GTC-CTG-GGT-ATA-

       10                                              20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-
```

30

His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-

40

Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-

50 (HindIII) 60

Ser Gln Ile Val Ser Phe Tyr Phe Lys Leu Glu Val Lys His-
AGC-CAA-ATT-GTC-TCC-TTT-TAC-TTC-AAG-CTT-GAA-GTA-AAA-CAT-
TCG-GTT-TAA-CAG-AGG-AAA-ATG-AAG-TTC-GAA-CTT-CAT-TTT-GTA-

(EcoRI) 70 (HincII)

Glu Phe Met Gly Pro Glu Thr Leu Cys Gly Ala Glu Leu Val
GAA-TTC-ATG-GGT-CCT-GAA-ACT-CTG-TGC-GGC-GCT-GAA-CTG-GTT-
CTT-AAG-TAC-CCA-GGA-CTT-TGA-GAC-ACG-CCG-CGA-CTT-GAC-CAA-

80 90

Asp Ala Leu Gln Phe Val Cys Gly Asp Arg Gly Phe Tyr Phe
GAC-GCT-CTG-CAA-TTT-GTA-TGT-GGT-GAT-CGT-GGT-TTC-TAC-TTC-
CTG-CGA-GAC-GTT-AAA-CAT-ACA-CCA-CTA-GCA-CCA-AAG-ATG-AAG-

100

Asn Lys Pro Thr Gly Tyr Gly Ser Ser Ser Arg Arg Ala Pro
AAC-AAA-CCG-ACC-GGC-TAT-GGC-TCC-AGC-TCT-CGT-CGC-GCA-CCG-
TTG-TTT-GGC-TGG-CCG-ATA-CCG-AGG-TCG-AGA-GCA-GCG-CGT-GGC-

110

Gln Thr Gly Ile Val Asp Glu Cys Cys Phe Arg Ser Cys Asp
CAG-ACT-GGT-ATC-GTA-GAC-GAA-TGC-TGT-TTT-CGT-TCT-TGC-GAT-
GTC-TGA-CCA-TAG-CAT-CTG-CTT-ACG-ACA-AAA-GCA-AGA-ACG-CTA-

120 130

Leu Arg Arg Leu Glu Met Tyr Cys Ala Pro Leu Lys Pro Ala

CTC-CGC-CGT-CTG-GAA-ATG-TAC-TGT-GCT-CCA-CTG-AAG-CCA-GCA-

GAG-GCG-GCA-GAC-CTT-TAC-ATG-ACA-CGA-GGT-GAC-TTC-GGT-CGT-

136 (BamHI)

Lys Ser Ala Stop stop

AAA-TCC-GCG-TGA-TAG-3'

TTT-AGG-CGC-ACT-ATC-CTAG-5' .

In this process, a plasmid from which a gene of some length including a rop gene is cut off is used. In this case, if the cut off is done too widely and in essentially necessary part of gene, for example, a gene including a terminator region is removed together with said rop gene, an artificially synthesized gene which has the same function may be re-inserted in place of said part of gene.

Suitable promoter gene may include a conventionally employed promoter gene (e.g. trp-promoter gene, etc.) as well as a synthetic promoter gene. Suitable examples of the promoter gene may be synthetic trp promoter I gene (107 bp) which was synthesized by the inventors of this invention and DNA sequences thereof are as follows.

(EcoRI*)

5'-AATTTGCCGACATCATAACGGTTCTGGCAAATATTCTGAAATGAGC-

3'- ACGGCTGTAGTATTGCCAAGACCGTTTATAAGACTTTACTCG-

TGTTGACAATTAATCATCGAACTAGTTAACTAGTACGCAAGTTCACGTAAA-

ACAACTGTTAATTAGTAGCTTGATCAATTGATCATGCGTTCAAGTGCATTT-

(EcoRI)

AAGGGTATCG-3'

TTCCCATAGCTTAA-5'

In this process, one or more than one fused IGF-I genes may be inserted consecutively to an expression plasmid to give expression plasmid from which a gene of some length including a rop gene is cut off.

The promoter gene and fused IGF-I gene can consecutively and circularly be linked with an adequate plasmid together, if desired, by using an adequate DNA fragment(s) (e.g. linker, other restriction site , etc.) in a conventional manner (e.g. digestion with restriction enzyme, phosphorylation using T4 polynucleotide kinase, ligation using T4 DNA ligase) to give an expression plasmid.

Suitable "Co1E1 plasmid" may include pBR322 and the like.

The expression plasmid can be inserted into a microorganism (host cell) by a conventional manner (e.g. transformation, microinjection, etc.) to give a transformant.

Suitable host organism may include Escherichia (hereinafter referred to as E. coli (e.g. E. coli HB101, etc.) and the like.

For production of fused IGF-I by the process of invention, thus obtained transformant comprising the expression plasmid is cultured in a nutrient medium.

The nutrient medium contains carbon source(s) (e.g. glucose, glycerine, mannitol, fructose, lactose, etc.) and inorganic or organic nitrogen source(s) (ammonium sulfate, ammonium chloride, hydrolysate of casein, yeast extract, polypeptone, bactotrypton, beef extracts, etc.). If desired, other nutritious sources [e.g. inorganic salts (e.g. sodium or potassium biphosphate, dipotassium hydrogen phosphate, magnesium chloride, magnesium sulfate, calcium chloride), vitamins(e.g. vitamin $B_1$), antibiotics(e.g. ampicillin), etc.] may be added to the medium.

The culture of transformant may generally be carried out at pH 5.5-8.5 (preferably pH 7-7.5) and 18-40 °C (preferably 25-38 °C) for 5-50 hours.

Since thus produced fused IGF-I generally exists in cells of the cultured transformant, the cells are collected by filtration or centrifuge, and cell wall and/or cell membrane thereof is destroyed in a conventional manner (e.g. treatment with super sonic waves and/or lysozyme , etc.) to give debris. From the debris, the fused IGF-I can be isolated and purified in a conventional manner as generally employed for the isolation and purification of natural or synthetic proteins [e.g. dissolution of protein with an appropriate solvent (e.g. 8M aqueous urea, 6M guanidine hydrochloride, etc.), dialysis, gel filtration, column chromatography, high performance liquid chromatography, etc.].

One of the suitable examples of thus obtained fused IGF-I may be "IGF-I fused with protein peptide LH", amino acid sequence of which is as follows.

```
 1                                      10
Cys-Tyr-Cys-Gln-Asp-Pro-Tyr-Val-Lys-Glu-Ala-Glu-Asn-Leu-

                       20
Lys-Lys-Tyr-Phe-Asn-Ala-Gly-His-Ser-Asp-Val-Ala-Asp-Asn-

   30                                               40
Gly-Thr-Leu-Phe-Leu-Gly-Ile-Leu-Lys-Asn-Trp-Lys-Glu-Glu-

                                 50
Ser-Asp-Arg-Lys-Ile-Met-Gln-Ser-Gln-Ile-Val-Ser-Phe-Tyr-

             60                                     70
Phe-Lys-Leu-Glu-Val-Lys-His-Glu-Phe-Met-Gly-Pro-Glu-Thr-

                                        80
Leu-Cys-Gly-Ala-Glu-Leu-Val-Asp-Ala-Leu-Gln-Phe-Val-Cys-

                     90
Gly-Asp-Arg-Gly-Phe-Tyr-Phe-Asn-Lys-Pro-Thr-Gly-Tyr-Gly-

   100                                         110
Ser-Ser-Ser-Arg-Arg-Ala-Pro-Gln-Thr-Gly-Ile-Val-Asp-Glu-

                                 120
Cys-Cys-Phe-Arg-Ser-Cys-Asp-Leu-Arg-Arg-Leu-Glu-Met-Tyr-

             130                   136
Cys-Ala-Pro-Leu-Lys-Pro-Ala-Lys-Ser-Ala .
```

The present elimination reaction can be conducted under mild conditions in a conventional solvent which does not adversely affect the reaction.

In the sequence in this specification A, G, C and T mean the formula:

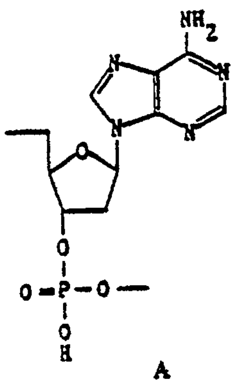
A
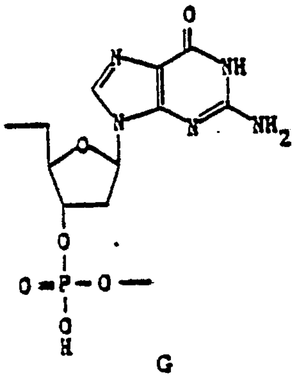
G
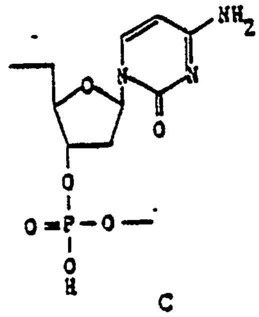
C
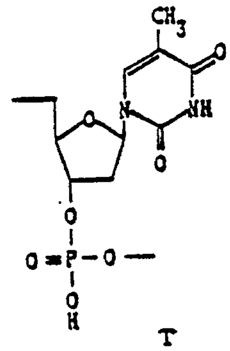
T

respectively, and
5′-terminal A, G, C and T mean the formula:

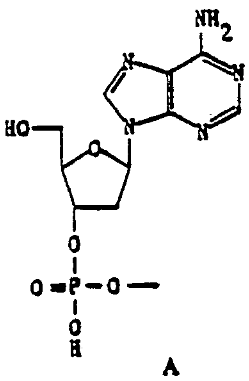
A
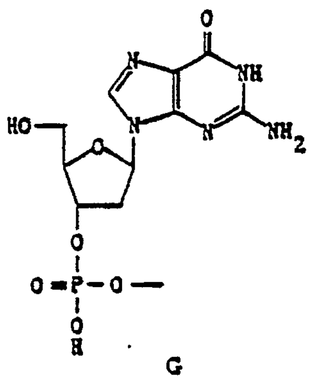
G
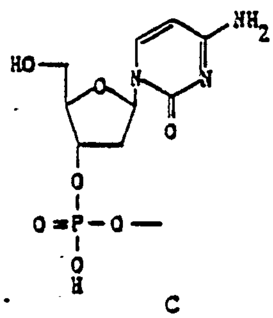
C
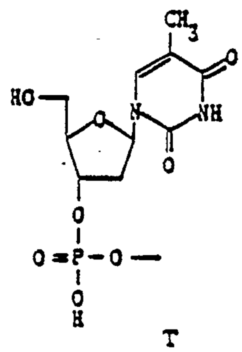
T

respectively, and 3′ terminal A, G, C and T mean the formula:

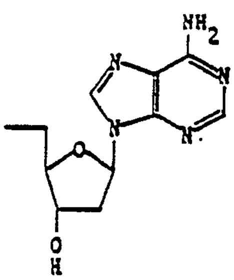
A
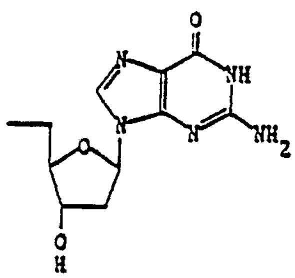
G
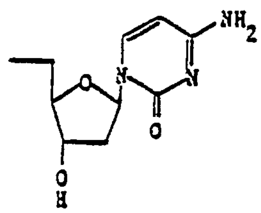
C
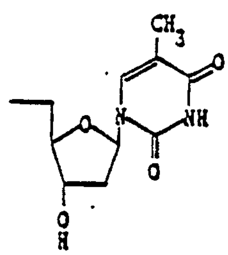
T

respectively.

The following examples are given for the purpose of illustrating the present invention.

## Example 1

Preparation of oligonucleotides

Following oligonucleotides were prepared by a conventional method.

(1) HOApApTpTpCpApTpGpGpGpTOH (A1)
(2) HOTpTpTpCpApGpGpApCpCpCpApTpGOH (A2)
(3) HOCpCpTpGpApApApCpTpCpTpGpTpGOH (B1)
(4) HOCpApGpCpGpCpCpGpCpApCpApGpApGOH (B2)

(5) HOCpGpGpCpGpCpTpGpApApCpTpGpGpTOH (C1)
(6) HOApGpApGpCpGpTpCpApApCpCpApGpTpTOH (C2)
(7) HOTpGpApCpGpCpTpCpTpGpCpApApTpTpTOH (D1)
(8) HOCpCpApCpApTpApCpApApApTpTpGpCOH (D2)
(9) HOGpTpApTpGpTpGpGpTpGpApTpCpGpTOH (E1)
(10) HOTpApGpApApApCpCpApCpGpApTpCpAOH (E2)
(11) HOGpGpTpTpTpCpTpApCpTpTpCpApApCOH (F1)
(12) HOGpGpTpCpGpGpTpTpTpGpTpTpGpApApGOH (F2)
(13) HOApApApCpCpGpApCpCpGpGpCpTpApTpGOH (G1)
(14) HOGpCpTpGpGpApGpCpCpApTpApGpCpCOH (G2)
(15) HOGpCpTpCpCpApGpCpTpCpTpCpGpTpCOH (H1)
(16) HOCpGpGpTpGpCpGpCpGpApCpGpApGpAOH (H2)
(17) HOGpCpGpCpApCpCpGpCpApGpApCpTpGOH (I1)
(18) HOCpTpApCpGpApTpApCpCpApGpTpCpTpGOH (I2)
(19) HOGpTpApTpCpGpTpApGpApCpGpApApTpGOH (J1)
(20) HOGpApApApApCpApGpCpApTpTpCpGpTOH (J2)
(21) HOCpTpGpTpTpTpTpCpGpTpTpCpTpTpGOH (K1)
(22) HOGpGpApGpApTpCpGpCpApApGpApApCOH (K2)
(23) HOCpGpApTpCpTpCpCpGpCpCpGpTpCpTOH (L1)
(24) HOTpApCpApTpTpTpCpCpApGpApCpGpGpCOH (L2)
(25) HOGpGpApApApTpGpTpApCpTpGpTpGpCpTOH (M1)
(26) HOTpTpCpApGpTpGpGpApGpCpApCpApGOH (M2)
(27) HOCpCpApCpTpGpApApGpCpCpApGpCpAOH (N1)
(28) HOGpCpGpGpApTpTpTpTpGpCpTpGpGpCOH (N2)
(29) HOApApApTpCpCpGpCpGpTpGpApTpApGOH (O1)
(30) HOGpApTpCpCpTpApTpCpApCOH (O2)
(31) HOApApTpTpCpApTpGpTpGpTpTOH (a1)
(32) HOApCpTpGpCpCpApGpGpApCpCpCpApTOH (a2)
(33) HoApTpGpTpApApApApGpApApGpCpApGOH (a3)
(34) HOTpGpGpCpApGpTpApApCpApCpApTpGOH (a4)
(35) HOTpTpTpApCpApTpApTpGpGpGpTpCpCOH (a5)
(36) HOApApGpGpTpTpTpTpCpTpGpCpTpTpCpTOH (a6)
(37) HOApApApApCpCpTpTpApApGpApApApTpAOH (b1)
(38) HOCpTpTpTpApApTpGpCpApGpGpTpCpAOH (b2)
(39) HOTpTpCpApGpApTpGpTpApGpCpGpGpAOH (b3)
(40) HOApTpTpApApApGpTpApTpTpTpCpTpTOH (b4)
(41) HOApTpCpTpGpApApTpGpApCpCpTpGpCOH (b5)
(42) HOTpTpCpCpApTpTpApTpCpCpGpCpTpApCOH (b6)
(43) HOTpApApTpGpGpApApCpTpCpTpTpTpTpCOH (c1)
(44) HOTpTpApGpGpCpApTpTpTpTpGpApApGOH (c2)
(45) HOApApTpTpGpGpApApApGpApGpGpApGOH (c3)
(46) HOTpGpCpCpTpApApGpApApApApGpApGOH (c4)
(47) HOTpCpCpApApTpTpCpTpTpCpApApApAOH (c5)
(48) HOCpTpGpTpCpApCpTpCpTpCpCpTpCpTpTOH (c6)
(49) HOApGpTpGpApCpApGpApApApApApTpAOH (d1)
(50) HOApTpGpCpApGpApGpCpCpApApApTpTOH (d2)
(51) HOGpTpCpTpCpCpTpTpTpTpApCpTpTOH (d3)
(52) HOCpTpCpTpGpCpApTpTpApTpTpTpTpTOH (d4)
(53) HOApGpGpApGpApCpApApTpTpTpGpGOH (d5)
(54) HOApApApGpCpTpTpGpApApGpTpApApAOH (d6)
(55) HOCpApApGpCpTpTpTpTpCpApApApApAOH (e1)
(56) HOCpTpTpTpApApGpGpApTpGpApCpCpAOH (e2)
(57) HOGpApGpCpApTpCpCpApApApApGpApGOH (e3)
(58) HOCpCpTpTpApApApGpTpTpTpTpTpGpAOH (e4)
(59) HOGpGpApTpGpCpTpCpTpGpGpTpCpApTOH (e5)
(60) HOTpGpTpGpTpApApTpGpApTpApGOH (ℓ1)
(61) HOTpApCpApCpApCpTpCpTpTpTpTOH (ℓ2)
(62) HOGpApTpCpCpTpApTpCpApTOH (ℓ3)

(63) HOApGpCpTpTpGpApApGpTpApApApApCpApTpGOH (m1)
(64) HOApApTpTpCpApTpGpTpTpTpTpApCpTpTpCpAOH (m2)
(65) HOApGpCpTpTpGpApApGpTpApTpGpGpGOH (LA)
(66) HOGpApCpCpCpCpApTpApCpTpTpCpAOH (LB)
(67) HOApApTpTpCpGpGpCpCpCpCpGpCpGpGOH (LC)
(68) HOGpApCpCpCpGpCpGpGpGpGpCpCpGOH (LD)
(69) HOApApTpTpTpGpCpCpGpApCpAOH (A)
(70) HOCpGpTpTpApTpGpApTpGpTpCpGpGpCpAOH (B)
(71) HOTpCpApTpApApCpGpGpTpTpCpTpGpGpCOH (C)
(72) HOGpApApTpApTpTpTpGpCpCpApGpApApCOH (D)
(73) HOApApApTpApTpTpCpTpGpApApApTpGpAOH (E)
(74) HOTpCpApApCpApGpCpTpCpApTpTpTpCpAOH (F)
(75) HOGpCpTpGpTpTpGpApCpApApTpTpApApTOH (G)
(76) HOGpTpTpCpGpApTpGpApTpTpApApTpTpGOH (H)
(77) HOCpApTpCpGpApApCpTpApGpTpTpApApCOH (I)
(78) HOGpCpGpTpApCpTpApGpTpTpApApCpTpAOH (J)
(79) HOTpApGpTpApCpGpCpApApGpTpTpCpApCOH (K)
(80) HOCpTpTpTpTpTpApCpGpTpGpApApCpTpTOH (L)
(81) HOGpTpApApApApApGpGpGpTpApTpCpGOH (M)
(82) HOApApTpTpCpGpApTpApCpCOH (N)
(83) HOApApTpTpCpApTpGpGpCpTOH (SA)
(84) HOGpGpTpTpGpTpApApGpApApCpTpTpCpTOH (SB)
(85) HOTpTpTpGpGpApApGpApCpTpTpTOH (SC)
(86) HOCpApCpTpTpCpGpTpGpTpTpGpApTpApGOH (SD)
(87) HOTpTpApCpApApCpCpApGpCpCpApTpGOH (SE)
(88) HOCpCpApApApApGpApApGpTpTpCOH (SF)
(89) HOCpGpApApGpTpGpApApApGpTpCpTpTOH (SG)
(90) HOGpApTpCpCpTpApTpCpApApCpAOH (SH)
(91) HOGpApTpCpCpTpCpGpApGpApTpCpApAOH (A')
(92) HOGpCpCpTpTpTpApApTpTpCpApTpCpTpCpGpApGOH (B')
(93) HOTpTpApApApGpGpCpTpCpCpTpTpTpTpGpGpAOH (C')
(94) HOApApApApApGpGpCpTpCpCpApApApApGpGpAOH (D')
(95) HOGpCpCpTpTpTpTpTpTpTpTpTpTpGOH (E')
(96) HOTpCpGpApCpApApApApAOH (F')
(97) HOGpApTpCpCpTpCpGpApGpCpTOH (G')
(98) HOGpTpTpTpApApTpCpApGpCpTpCpGpApGOH (H')
(99) HOGpApTpTpApApApCpCpGpApApTpCpApAOH (I')
(100) HOGpCpCpTpTpTpApApTpTpGpApTpTpCpGOH (J')
(101) HOGpCpCpTpTpTpTpTpTpTpTpTpTOH (K'')
(102) HOTpCpTpCpCpApApApApAOH (L')
(103) HOGpGpApGpApCpApApCpGOH (M')
(104) HOTpCpGpApCpGpTpTpGOH (N')

In the above oligonucleotides, following abbreviations are used.

Ap, Gp, Cp and Tp mean the formula:

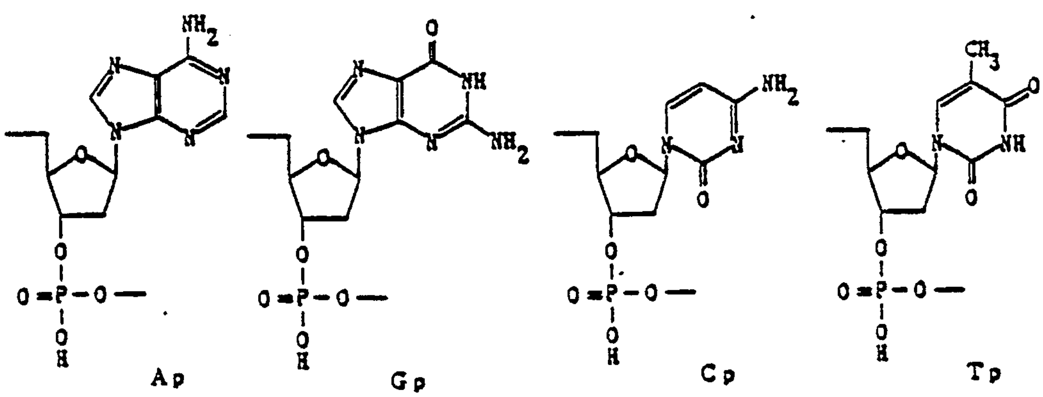

Ap Gp Cp Tp

respectively, and
3′-teminal A, G, C and T mean the formula:

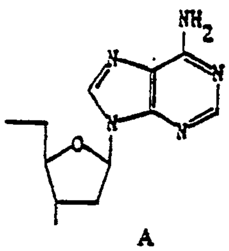
A
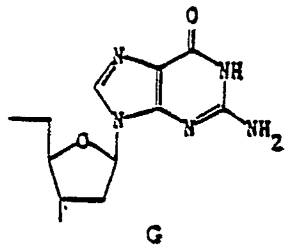
G
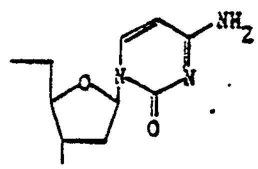
C
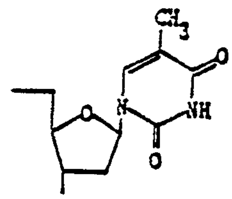
T

respectively.

Example 2

Preparation of IGF-I gene:

Aliquots of each oligonucleotides (A1-O2) (0.4 nM) were phosphorylated with 4 units of T4 polynucleotide kinase [made by Bethesda Research Laborabories (BRL)] in 100 µl of a solution containing 74 mM Tris-HC1 (pH 7.6), 10 mM dithiothreitol (hereinafter referred to as DTT), 1.6 mM mercaptoethanol, 10 mM $MgC1_2$ and 0.5 mM ATP for 20 minutes at 37° C. After the reaction was completed, the enzyme in the reaction mixture was deactivated by incubation at 100° C for 5 minutes. Ligation of the phosphorylated oligonucleotides was carried out as shown in Fig. 1 to give firstly fragment ten blocks and ultimately the IGF-I gene for cloning. Ligations were carried out with T4 DNA ligase (7 units) in a solution containing 100 mM ATP (0. 5 µl) for 23 hours at 4° C (standard conditions). The ligation products of oligonucleotides in each step were identified by staining with ethidium bromide following electroelution on a 2-16% gradient polyacrylamide gel electrophoresis (hereinafter referred to as PAGE) in tris-acetate-EDTA buffer.

Example 3

Molecular cloning of IGF-I gene:

Plasmid pBR322 was digested with BamHI and EcoRI restriction endonucleases. Reaction was terminated by heating at 65° C for 5 minutes and the fragments separated by electrophoresis on a 0.5% agarose gel. The 3985 bp large fragment from pBR322 was recovered and ligated with T4 DNA ligase for 18 hours at 12° C to the 224 bp IGF-I gene. The ligated mixture was transformed into E. coli HB101 by Kushner's method and ampicillin resistant transformants were selected on the plate containing tetracycline (25 µg/ml). Pasmid DNA isolated from one of five clones resistant to ampicillin ($^{r}$Amp) and sensitive to tetracycline ($^{s}$Tet) was digested with EcoRI and BamHI and compared with appropriate size markers. The expected 224 bp IGF-I fragment was generated. This plasmid which was characterized by complete nucleotide sequencing of the IGF-I gene was named pSdM1 and was used for the construction of expression plasmid. This process is shown in Fig. 2.

Example 4

Preparation of synthetic trp promoter II gene:

Each oligonucleotides (B to SH) of block I, II, III and IV were phosphorylated with T4 polynucleotide kinase and then ligated with T4 DNA ligase as described above. These blocks (I to IV) and unphosphorylated oligonucleotides (A and SH) were condensed successively. The last ligation product was purified by preparative 7.5% PAGE to give synthetic trp promoter II gene (which is synthetic trp promoter I gene with an adequate DNA fragment) (163 bp). This process is shown in Fig. 3. Thus obtained synthetic trp promoter II gene (163 bp) is as follows:

```
        EcoRI*
5'- AATTTGCCGACATCATAACGGTTCTGGCAAATATTCTGAAATGAGC-
3'-      ACGGCTGTAGTATTGCCAAGACCGTTTATAAGACTTTACTCG-

                                   HpaI
TGTTGACAATTAATCATCGAACTAGTTAACTAGTACGCAAGTTCACGTAAA-
ACAACTGTTAATTAGTAGCTTGATCAATTGATCATGCGTTCAAGTGCATTT-

             EcoRI
AAGGGTATCGAATTCATGGCTGGTTGTAAGAACTTCTTTTGGAAGACTTTC-
TTCCCATAGCTTAAGTACCGACCAACATTCTTGAAGAAAACCTTCTGAAAG-

                BamHI
ACTTCGTGTTGATAG-3'
TGAAGCACAACTATCCTAG-5'
```

Example 5

Molecular cloning of the sythetic trp promoter II gene:

Trp promoter II gene constructed in Example 4 was ligated with EcoRI, BamHI fragment of pBR322 and then E. coli HB101 was transformed with the ligation product. The plasmid obtained from the transformant of $^{r}$Amp and $^{s}$Tet was digested with HpaI to confirm a band (4.1 kbp), and then digested with BamHI to confirm a band of 90 bp on PAGE. Moreover, the fragment of 56 bp by EcoRI-BamHI digestion was confirmed by the comparison with size marker on PAGE. This plasmid was named pTrpEB7 and used for the construction of expression plasmid. This process is shown in Fig. 4.

Example 6

Preparation of protein peptide LH gene:

Aliquots of each oligonucleotides (a2-ℓ2) (0.4 nM) were phosphorylated with 2.5 units of T4 polynucleotide kinase in 40 µl of a solution containing 50 mM Tris-HC1 (pH 7.6), 20 mM DTT, 50 µg/ml bovine serum albumin (hereinafter referred to as BSA), 1 mM spermidine, 10 mM $MgC1_2$ and 2 mM ATP for 3 hours at 37°C. After the reaction was completed, the enzyme in the reaction mixture was deactivated by incubation at 100°C for 5 minutes. Ligation of the phosphorylated oligonucleotides and two oligonucleotides (a1 and ℓ3) was carried out as shown in Fig. 5 to give firstly fragment six blocks and ultimately protein peptide LH gene (236 bp) for cloning. Ligation was carried out with T4 DNA ligase (5 units) for 5 hours at 16°C. The ligation products of oligonucleotides in each step were identified by staining with ethidium bromide following electroelution on a 2-16% gradient PAGE in Tris-acetate-EDTA buffer. This process is shown in Fig. 5.

Example 7

Molecular cloning of protein peptide LH gene:

Protein peptide LH gene (236 bp) which synthesized as above was inserted into pBR322 by a similar

manner to that of Example 3. The plasmid (pLH107) obtained from E. coli HB101 transformant was characterized by restriction enzyme analysis to have protein peptide LH (236bp). This process is shown in Fig. 6

Example 8

Construction of protein peptide LH expression plasmid (pLHtrp):

Plasmid pTrpEB7 prepared in Example 5 was digested with EcoRI and BamHI to give a large fragment (4.1 kbp) by preparative agarose gel electrophoresis. This fragment was ligated with protein peptide LH gene prepared from a plasmid pLH107 by EcoRI and BamHI digestion. The ligated mixture was transformed into E. coli HB101 to give ampicillin resistant and tetracycline sensitive transformants. The plasmid (pLHtrp) obtained from the transformant was digested with EcoRI and BamHI to confirm protein peptide LH gene (236 bp) on 7.5% PAGE. This process is shown in Fig. 7.

Example 9

Construction of IGF-I expression plasmid pLHSdMmtrp:

Plasmid pSdM1 was digested with EcoRI and BamHI to give IGF-I gene (224 bp). On the other hand, oligonucleotide (m2) prepared in Example 1 was phosphorylated with T4 polynucleotide kinase as described in Example 2. The phosphorylated oligonucleotide, oligonucleotide m1 prepared in Example 1 and IGF-I gene (224 bp) were mixed and treated with T4 ligase for 20 hours at 4° C. The ligation mixture was digested with BamHI and then purified by preparative PAGE to give IGF-I gene with linker (242 bp). The gene (242 bp) was ligated with the large fragment obtained from pLHtrp by HindIII-BamHI digestion, and then the ligation mixture was transformed into E. coli HB101. The E. coli HB101 containing plasmid pLHSdMmrtrp was named E. coli F-6 and deposited with Fermentation Research Institute Agency of Industrial Science and Technology (1-3, Higashi 1 chome Yatabe-machi Tsukuba-gun Ibaraki-ken 305, Japan) under deposit number of FERM P-7848 on September 17, 1984, and then converted to Budapest Treaty route of the same depository on February 28, 1985 under the new deposit number of FERM BP-729. The plasmid (pLHSdMmrtrp) obtained from the transformant was digested with EcoRI and BamHI (198, 224 bp), HindIII and BamHI (242 bp), HpaI-BamHI (456 bp) to confirm the synthetic trp promoter I, protein peptide LH and IGF-I gene on 7.5% PAGE. This process is shown in Fig. 8.

Example 10

Construction of plasmid pUC-SS1

The expression plasmid of IGF-I fused with protein peptide LH (pLHSdMmrtrp) (100 µg) was digested with HpaI (180 units) at 37° C for 1 hour in HpaI digestion buffer. After a detection of the complete digestion on 0.8% agarose gel, 1M NaC1 and PstI (180 units) were added to the mixture, and the mixture was incubated at 37° C for 1 hour to give two fragments (3.7 kbp and 0.8 kbp). The large fragment (3.7 kbp) was separated on 0.8% agarose gel, purified by DEAE-TOYOPEARL 650M (anion exchange resin having diethylaminoethyl groups, made by Toyo Soda Manufacturing Co., Ltd.) column chromatography, followed by ethanol precipitation to give a HpaI-PstI digested fragment (3.7 kbp) (40 µg). The fragment (35 µg) was partially digested with HincII (63 units) in HincII buffer at 37° C for 18 minutes to give six fragments (3690, 3417, 2958, 732, 459 and 273 bp). The fragment (2 µg) of 732 bp was purified by preparative 0.8% agarose gel electrophoresis and DEAE TOYOPEARL 650M column chromatography.

On the other hand, plasmid pUC9 (purchased from Pharmacia) (10 µg) was digested with HincII (120 units) at 37° C for 1 hour to give a pUC9 HincII linear fragment (2 µg). This DNA (1.9 µg) was incubated with calf intestinal alkaline phosphatase (hereinafter referred to as CIP) (purchased from Boehringer Mannheim) (20 units) for 30 minutes at 37° C and then with additional CIP (20 units) for 30 minutes at 37° C. After inactivation of the enzyme by heating for 15 minutes at 65° C, the DNA was purified by phenolchloroform extraction, followed by Sephadex G-50 superfine spun column chromatography and ethanol precipitation to give 1.5 µg of dephosphorylated, HincII digested linear fragment of pUC9. The DNA (1.2 µg) was ligated with HpaI-HincII digested linear fragment (732 bp) prepared above containing IGF-I fused with protein peptide LH gene in the presence of T4 DNA ligase (8 units) and 2 mM ATP in ligation buffer for 20 hours at 16° C. The ligation mixture was transformed into E. coli MM 294 to give many ampicillin resistant

colonies. One of the 22 colonies was the desired plasmid pUC-SS1 which was confirmed by restriction endonuclease analysis with PstI (3.4 kbp) and BamHI (2.9 kbp and 0.45 kbp). This process is shown in Fig. 9 and Fig. 10.

Example 11

Construction of expression plasmid pLS-T2 and pLS-T3.

Plasmid pLHSdMmtrp (50 μg) was digested with BamHI (120 units) for 1 hour at 37°C in BamHI digestion buffer, followed by preparative 0.8% agarose gel electrophoresis to give 20 μg of linear fragment (4.5 kbp). This DNA (16 μg) was treated with CIP to afford dephosphorylated, BamHI digested linear fragment of pLHSdMmtrp (4 μg).

On the other hand pUC-SS1 (10 μg) was digested with BamHI (60 units) to give two fragments (2.6 kbp and 464 bp). The small fragment (464 bp) was purified by preparative 0.8% agarose gel electrophoresis. The obtained BamHI digested fragment (464 bp) (36 ng) and the above dephosphorylated, BamHI digested linear fragment of pLHSdMmtrp (200 ng) were incubated in the presence of T4 DNA ligase (4 units) for 20 hours at 16°C. E. coli HB101 was transformed with the ligation mixture to give the ampicillin resistant colonies. Seven of twelve colonies were plasmid having two cystron IGF-I fused with protein peptide LH gene (which is named pLS-T2) and one of them was plasmid having three cystron IGF-I gene fused with protein peptide LH gene (which is named pLS-T2), which were confirmed by restriction endonuclease analysis (pLS-T2: PstI-PvuII (1.5, 3.4 kbp), EcoRI (198, 266 bp), PstI-SalI (3.0, 2.0 kbp) HpaI (4.98 kbp); pLS-T3: PstI-PvuII (1.5, 3.9 kbp), EcoRI (198, 266 bp), PstI-SalI (3.0, 2.5 kbp), HpaI (5.45 kbp). This process is shown in Fig. 11 and Fig. 12 [Fig. 12(a) and Fig. 12(b)].

Example 12

Preparation of terminator S gene:

Each oligonucleotide (B', C', D' and E') were phosphorylated with T4 polynucleotide kinase and then treated with T4 DNA ligase as described in Example 2. The ligation mixture and two oligonucleotides (A' and F') were mixed and treated with T4 DNA ligase to give a ligation product. This process is shown in Fig. 13. The terminator S gene is as follows:

```
GATCCTCGAGATCAATTAAAGGCTCCTTTTGGAGCCTTTTTTTTTTG
    GAGCTCTACTTAATTTCCGAGGAAAACCTCGGAAAAAAAAAACAGCT
```

Example 13

Molecular cloning of terminator S gene:

The ligation product was purified by preparative PAGE and mixed with the large fragment of pBR322 by BamHI-SalI digestion. Ligation of the mixture was carried out with T4 ligase under the standard condition. The ligation mixture was transformed into E. coli HB101 by Kushner's method and ampicillin resistant transformants were selected on the plate containing tetracycline. Plasmid DNA isolated from a clone resistant to ampicillin and sensitive to tetracycline was digested with AvaI to show 817 bp fragment and with BamHI-SalI to confirm terminator S gene (47 bp). The plasmid was named pTerS21 and used for the construction of expression plasmid. This process is shown in Fig. 14.

Example 14 Construction of pLHSdMmTer:

Plasmid pTerS21 (20 μg) was digested with PstI (40 units) and BamHI (40 units) for 1 hour at 37°C. The long fragment (3 kbp) was purified by preparative 1.5% agarose gel electrophoresis, followed DEAE TOYOPEARL 650M column chromatography to give the fragment (2 μg) containing terminator S gene.

On the other hand, plasmid pLS-T2 (20 μg) was digested with PstI and BamHI to give the PstI-BamHI

fragment (2 µg) of 1.3 kbp. This fragment (1.3 kbp, 200 ng) containing fused IGF-I gene was ligated with the above fragment (3 kbp, 200 ng) in the presence of T4 DNA ligase (1 µl) for 2 hours at 16 °C. The ligation mixture was transformed into E. coli DH1 to give ampicillin resistant colonies. The plasmid obtained from the transformant was named pLHSdMmTer and was confirmed by restriction endonuclease analysis [XhoI (4.3 kbp), PstI-HindIII (3.3 kbp and 1.0 kbp)]. This process is shown in Fig. 15.

Example 15

Construction of expression plasmid pLS-D1:

Plasmid pLHSdMmTer (50 µg) was digested with SalI (80 units) and PvuII (80 units)to give two fragments (2.9 kbp and 1.4 kbp). After the separation and purification, the SalI-PvuII fragment (2.9 kbp) (1 µg) was incubated with DNA polymerase I (large fragment) (purchased from BRL) (2 units) and 0.1 mM 4dNTPs (equal molar dATP, dCTP, dGTP and TTP) in Nick translation buffer for 1 hour at 16 °C. After inactivation of the enzyme by heating for 5 minutes at 70 °C, the DNA was purified by preparative 0.8% agarose gel electrophoresis. The recovered DNA (200 ng) was treated with T4 DNA ligase and 2 mM ATP in ligation buffer for 20 hours at 16 °C. E. coli HB101 was transformed into the ligation mixture to give ampicillin resistant transformants. The plasmid obtained from the transformant was named pLS-D1. The E. coli HB101 containing plasmid pLS-D1 was named E. coli F-12. The plasmid (pLS-D1) obtained above from the transformant was confirmed by restriction endonuclease analysis [(Pst-BamHI (1.3 kbp), HpaI-BamHI (456 bp)]. This process is shown in Fig. 16.

Example 16

Construction of expression plasmid pLS-D2:

Plasmid pLS-D1 (10 µg) was digested with BamHI (30 units) to give a linear fragment (5 µg). The DNA (2.5 µg) was treated with CIP to give a dephosphorylated BamHI pLS-D1 fragment (2 µg). The dephosphorylated BamHI fragment (200 ng) was ligated with the BamHI fragment (464 bp) containing fused IGF-I gene prepared in Example 11 in the presence of T4 DNA ligase and 2 mM ATP for 20 hours at 16 °C. E. coli HB101 was transformed with the ligation mixture. The plasmid obtained from the ampicillin resistant transformant was named pLS-D2. The E. coli HB101 containing plasmid pLS-D2 is named E. coli F-13. The plasmid (pLS-D2) obtained from the transformant was confirmed by the digestion with PstI (3.3 kbp), PstI-BamHI (1.6 kbp, 1.3 kbp and 464 bp) and BamHI (2.9 kbp, 464 bp). This process is shown in Fig. 17.

Example 17

Construction of expression plasmid pLS-DD1:

Plasmid pLS-D1 (40 µg) was digested with PstI (43 unites) to give a linear fragment (20 µg). The DNA was treated with CP to obtain a dephosphorylated PstI digested pLS-D1 (20 µg). Plasmid pLS-D2 (40 µg) was digested with PstI (24 units). The obtained PstI digested pLS-D2 (1 µg) and the dephosphorylated PstI digested pLS-D1 (5 µg) were incubated in the presence of T4 DNA ligase (1 µl) and 2 mM ATP for 20 hours at 16 °C. The ligation mixture was transformed into E. coli HB101 to give ampicillin resistant transformants. The plasmid obtained from the transformant was named pLS-DD1. The E. coli HB101 containing plasmid pLS-DD1 was named E. coli F-14. The plasmid obtained from the transformant was confirmed by restriction endonuclease analysis (SalI (3.3 kbp and 2.9 kbp), HpaI (3.3 kbp and 2.9 kbp) and HpaI-SalI (2.4 kbp, 503 bp and 967 bp). This process is shown in Fig. 18 [Fig. 18(a), Fig. 18(b) and Fig. 18-(c)].

Example 18

Expression of a gene coding for IGF-I fused with protein peptide LH in E. coli F-12:

An overnight culture of E. coli F-12 (which is E. coli HB101 containing plasmid pLS-D1) in L broth containing 50µg/ml ampicillin was diluted 1:20 in M9 medium containing 0.2% glucose, 0.5% casamino acid (acid-hydrolyzed casein), 50 µg/ml vitamin B1 and 25 µg/ml ampicillin. β-Indole acrylic acid was added to a final concentration of 10µg/ml when $A_{600}$ was about 0.5. Then the cells were incubated and aliquots (20 ml

of culture broth) were collected by centrifugation (7 krpm, 4° C, 5 minutes).

Example 19

Expression of a gene coding for IGF-I fused with protein peptide LH in E. coli F-13:

An overnight culture of E. coli F-13 (which is E. coli HB101 containing plasmid pLS-D2) in L broth containing 50µg/ml ampicillin was diluted 1:20 in M9 medium containing 0.2% glucose, 0.5% casamino acid (acid-hydrolyzed casein), 50 µg/ml vitamin B1 and 25 µg/ml ampicillin. β-Indole acrylic acid was added to a final concentration of 10µg/ml when $A_{600}$ was about 0.5. Then the cells were incubated and aliquots (20 ml of culture broth) were collected by centrifugation (7 krpm, 4° C, 5 minutes).

Example 20

Expression of a gene coding for IGF-I fused with protein peptide LH in E. coli F-14:

An overnight culture of E. coli F-14 (which is E. coli HB101 containing plasmid pLS-DD1) in L broth containing 50µg/ml ampicillin was diluted 1:20 in M9 medium containing 0.2% glucose, 0.5% casamino acid (acid-hydrolyzed casein), 50 µg/ml vitamin B1 and 25 µg/ml ampicillin. β-Indole acrylic acid was added to a final concentration of 10µg/ml when $A_{600}$ was about 0.5. Then the cells were incubated and aliquots (20 ml of culture broth) were collected by centrifugation (7 krpm, 4° C, 5 minutes).

Example 21

Isolation and purification of IGF-I fused with protein peptide LH:

E. coli F-12 was cultured for 4 hours after induction and collected by centrifugation (14 krpm, 4° C). Wet cell paste (120 g: from 20 liter culture broth) was suspended in 300 ml of 10 mM phosphate buffer saline (hereinafter referred to as PBS) - 10 mM EDTA (pH 8.0) and freezed at -80° C. The mixture was thawed and added to 50 ml of 0.5 M EDTA and 50 ml of 10 mg/ml lisozyme solution. After stirring for 1 hour at 0° C, the mixture was homogenized. The cell debris was suspended in 2 liter of 25 mM PBS-10 mM EDTA-0.5% sodium sarcosyl (pH 8.0), and then the mixture was homogenized. After stirring for 1 hour at 0° C, the mixture was centrifuged at 7,000 rpm for 35 minutes at 4° C. The pellet was suspended in 1 liter of 10 mM PBS-10 mM EDTA (pH 8.0). The mixture was homogenized and centrifuged above the same method. The pellet was dissolved in 200 ml of 6 M guanidine hydrochloride-100 mM Tris chloride-10mM EDTA-100 mM DTT (pH 8.0), and centrifuged at 40 krpm for 1 hour at 20° C. The supernatant was collected and applied to a Sephacryl S300 superfine column (5.0 × 86.6 cm; 1700 ml resin) equilibrated with 0.1 M Tris-HC1 (pH 8.0)/8 M urea and 10 mM 2-mercaptoethanol. Elution was carried out at 4° C with equilibration buffer, at a flow rate of 0.6 ml/min. Sephacryl S 300 chromatography was conducted and fractions of 35 ml were collected. Sephacryl S300 chromatography was conducted. Assays were performed immediately following fractionation for all chromatography steps. Active fraction were collected and the pooled fraction of 500 ml was dialyzed for 3 hours at room temperature against 16 liters of 1 M acetic acid aqueous solution and then overnight against 16 liters of fresh 1 M acetic acid aqueous solution. The fraction dialyzed was lyophilized to give IGF-I fused with protein peptide LH (1.30 g) which contains a desired component. The IGF-I fused with protein peptide LH shows a band at the position of molecular weight 15,500 on 15% SDS PAGE (sodium dodecylsulfate polyacrylamide gel electrophoresis).

Example 22

The content of IGF-I fused with protein peptide LH in culture fluid was assayed using radioimmunoassay (hereinafter referred to as RIA) and calculated as the content of IGF-I

Radioimmunoassay of IGF-I was followed the method established by N. Yanaihara [N. Yanaihara et al: Peptide Hormones in Pancreas 3, 28(1983)].

Method:

With 0.1 ml of the above sample or standard sample (IGF-I fragment (26-46)) sample buffer [0.5% BSA in 0.01M PBS, 0.025 M EDTA (pH 7.4) (0.4 ml)], rabbit antiserum (0.1 ml) of IGF-I (26-46) and $^{125}$I-IGF-I (26-

46) (0.1 ml) were mixed. The mixture was allowed to stand for 48 hours at 4° C, and then added with rabbit serum (0.1 ml), rabbit γ-globulin antiserum (0.1 ml) and 5% PEG6000 (0.9 ml). After standing for additional 2 hours at 4° C the pellet was collected by centrifugation (3 krpm, 4° C, 30 minutes), and measured radio activity by γ-counter. The content of IGF-I was calculated from this radio activity.

Results:

The culture fluid (20 ml) (culture broth of E. coli F-12, E. coli F-13 and E. coli F-14 in M9-broth) which was prepared by the method described in Example 18, Example 19 and Example 20, respectively, was centrifuged at 7,000 rpm. The obtained cells were suspended in 8M urea, 10 mM EDTA (pH 8.0) (2ml) and then opened by sonication. The suspended solution was centrifuged (18,000 rpm, 30 minutes, 4° C), the supernatant was diluted with 0.5% BSA, 10 mM PBS, 25 mM EDTA to use as RIA and HPLC (high performance liquid chromatography) sample.

The content of IGF-I was compared to that of culture fluid similarly prepared using E. coli F-6 which contains expression plasmid pLHSdMmtrp (which was prepared by the method described in European Patent Application No. 85103060.1 filed on March 16, 1985).

| Expression plasmid | 5 hours (RIA μg/ml culture) |
|---|---|
| pLHSdMmtrp | 15.0 (4 hour) |
| pLS-D1 | 21.7 |
| pLS-D2 | 25.1 |
| pLS-DD1 | 32.2 |

Example 23

Elimination of protein peptide LH from fused IGF-I with cyanogen bromide:

The fused IGF-I (225 mg) obtained by the above-procedure was dissolved in 36 ml of 60% formic acid. Cyanogen bromide (36 mg) was added and the mixture was allowed to react for 3 hours below 25° C with stirring. After addition of 234 ml of distilled water, formic acid and cyanogen bromide were removed by lyophylization. The residue was dissolved in 36 ml of 1M Tris-HCl (pH 8.0)/8 M urea and 50 mM 2-mercaptoethanol. The resulting solution was dialyzed twice for 3 hours at room temperature against 400 ml of 0.01 M ammonium acetate (pH 4.6)/8 M urea and 50 mM 2-mercaptoethanol (Buffer A) and then overnight against 400 ml of fresh Buffer A. The solution dialyzed was applied to a cationic ion exchange resin CM 52 column (1.6 × 7.5 cm; 15 ml resin) equilibrated with Buffer A. The column was washed with Buffer A (60 ml) at room temperature at a flow rate of u.25 ml/min and eluted with a linear gradient from Buffer A (120 ml) to 0.2 M ammonium acetate/8 M urea and 50 mM 2-mercaptoethanol (120 ml). Fractions (from No. 57 to No. 100) of 2.9 ml were collected.

High performance liquid chromatography:

The pooled fraction obtained above was applied.
column: Beckman Ultrapore RPSC (trademark: made by Beckman) (4.6 × 75 mm)
flow rate: 1ml/min
elution: linear gadient from 10% to 60 % acetonitrile in 0.01 M trifluoroacetic acid over 50 minutes.

The chromatography was repeated 15 times and fractions containing reduced IGF-I were collected and the main peak with a retention time of 29.32 minutes corresponds to reduced IGF-I. Thus reduced IGF-I was obtained about 2.4 mg by the procedures described above. The reduced IGF-I was converted to oxidize IGF-I by usual manner of refolding. The IGF-I was supperimposed with authentic IGF-I gifted by Dr. Humbel on HPLC.

Amino acid analysis and sequence analysis of IGF-I:

The amino acid composition of IGF-I was obtained using a Walters amino acid analysis system. The amino acid sequence of IGF-I was determined by the combination of DIBITC method (modified Edman's method) [J. Y. Chang et al: Biochem. J., 153, 607(1976), Biochim. Biophys. Acta., 578, 188(1979)] and carboxypeptidase method as shown in Fig. 19.

## Claims

## Claims for the following Contracting States : BE, CH, DE, FR, GB, IT, LI, LU, NL, SE

1. A plasmid pLS-D1 which is obtainable by
   (a) isolating plasmid pLHSdMmtrp from E. coli F-6 FERM BP-729,
   (b) digesting the plasmid pLHSdMmtrp with HpaI end PstI to give HpaI-PstI digested fragment (3.7 kbp),
   (c) digesting partially the fragment (3.7 kbp) with HincII to give the HpaI-HincII digested fragment (732 bp),
   (d) digesting plasmid pUC9 with HincII,
   (e) dephosphorylating the resultant HincII digested fragment with calf intestinal alkaline phosphatase (CIP),
   (f) ligating the HincII digested fragment of pUC9 with the above HpaI-HincII digested fragment (732 bp) to give plasmid pUC-SS1,
   (g) digesting the plasmid pLHSdMmtrp with BamHI to give BamHI digested fragment (4.5 kbp),
   (h) dephosphorylating the fragment (4.5 kbp) with CIP,
   (i) digesting plasmid pUC-SS1 with BamHI to give BamHI digested fragment (464 bp),
   (j) ligating the BamHI digested fragment (464 bp) with the above dephosphorylated fragment (4.5 kbp) to give plasmid pLS-T2 which is composed of two PstI-PvuII digestible fragments (1.5 kbp and 3.4 kbp),
   (k) ligating the terminator S gene of the following DNA sequence:

   GATCCTCGAGATCAATTAAAGGCTCCTTTTGGAGCCTTTTTTTTTTG
   GAGCTCTACTTAATTTCCGAGGAAAACCTCGGAAAAAAAAAACAGCT

   with the large BamHI-SalI digested linear fragment of pBR322 to give plasmid pTerS21,
   (l) digesting the plasmid pTerS21 with PstI and BamHI to give PstI-BamHI digested fragment (3 kbp),
   (m) digesting the plasmid pLS-T2 with PstI and BamHI to give PstI-BamHI digested fragment (1.3 kbp),
   (n) ligating the PstI-BamHI digested fragment (1.3 kbp) with the above PstI-BamHI digested fragment (3 kbp) to give plasmid pLHSdMmTer which is composed of pstI-Hind III digestible fragments (3.3 kbp and 1.0 kbp),
   (o) digesting the plasmid pLHSdMmTer with SalI and PvuII to give SalI-PvuII digested fragment (2.9 kbp),
   (p) treating the SalI-PvuII digested fragment (2.9 kbp) with DNA polymerase (large fragment) and dNTPs (dATP, dCTP, dGTP and TTP) to give a linear fragment having two flush end, and
   (q) self-ligating the linear fragment to give plasmid pLS-D1.

2. A plasmid pLS-D2 which is obtainable by
   (a) digesting the plasmid pLS-D1 of claim 1 with BamHI to give BamHI digested pLS-D1 fragment,
   (b) dephosphorlating the BamHI digested pLS-D1 fragment with CIP,

(c) isolating plasmid pLHSdMmtrp from E. Coli F-6 FERM BP-729,
(d) digesting the plasmid pLHSdMmtrp with HpaI and PstI to give HpAI-PstI digested fragment (3.7 kbp),
(e) digesting partially the fragment (3.7 kbp) with HincII to give the HpaI-HincII digested fragment (732 bp),
(f) digesting plasmid pUC9 with HincII,
(g) dephosphorylating the resultant HincII digested fragment with calf intestinal alkaline phosphatase (CIP),
(h) ligating the HincII digested fragment of pUC9 with the above HpaI-HincII digested fragment (732 bp) to give plasmid pUC-SS1,
(i) digesting plasmid pUC-SS1 with BamHI to give BamHI digested pUC-SS1 fragment (464 bp), and
(j) ligating the BamHI digested pUC-SS1 fragment (464 bp) with the dephosphorylated and BamHI digested pLS-D1 fragment to give plasmid pLS-D2.

**3.** A plasmid pLS-DD1 which is obtainable by
(a) digesting the plasmid pLS-D1 of claim 1 with PstI to give PstI digested pLS-D1 fragment,
(b) dephosphorylating the PstI digested pLS-D1 fragment,
(c) digesting the plasmid pLS-D2 of claim 2 with PstI to give Pst I digested pLS-D2 fragment, and
(d) ligating the PstI digested pLS-D2 fragment with the dephosphorylated and PstI digested pLS-D1 fragment to give plasmid pLS-DD1

**4.** E. coli transformed with the plasmid pLS-D1 of claim 1.

**5.** E. coli transformed with the plasmid pLS-D2 of claim 2.

**6.** E. coli transformed with the plasmid pLS-DD1 of claim 3

**7.** A process for preparing IGF-IS fused with a protective peptide by culturing a E. coli transformed with the expression plasmid pLS-D1 of claim 1.

**8.** A process for preparing IGF-I fused with a Protective peptide by culturing a E. coli transformed with the expression plasmid pLS-D2 of claim 2.

**9.** A process for preparing IGF-I fused with a protective peptide by culturing a E. coli transformed with the expression plasmid pLS-DD1 of claim 3.

**Claims for the following Contracting States : AT, ES, GR**

**1.** A process for preparing the plasmid pLS-D1 comprising the followings steps:
(a) isolating plasmid pLHSdMmtrp from E. coli F-6 FERM BP-729,
(b) digesting the plasmid pLHSdMmtrp with HpaI and PstI to give HpaI-PstI digested fragment (3.7 kbp),
(c) digesting partially the fragment (3.7 kbp) with HincII to give the HpaI-HincII digested fragment (732 bp),
(d) digesting plasmid pUC9 with HincII,
(e) dephosphorylating the resultant HincII digested fragment with calf intestinal alkaline phosphatase (CIP),
(f) ligating the HincII digested fragment of pUC9 with the above HpaI-HincII digested fragment (732 bp) to give plasmid pUC-SS1,
g) digesting the plasmid pLHSdMmtrp with BamHI to give BamHI digested fragment (4.5 kbp),
(h) dephosphorylating the fragment (4.5 kbp) with CIP,
(i) digesting plasmid pUC-SS1 with BamHI to give BamHI digested fragment (464 bp),
(j) ligating the BamHI digested fragment (464 bp) with the above dephosphorylated fragment (4.5 kbp) to give plasmid pLS-T2 which is composed of two Pst1-PvuII digestible fragments (1.5 kbp and 3.4 kbp),
(k) ligating the terminator S gene of the following DNA sequence:

GATCCTCGAGATCAATTAAAGGCTCCTTTTGGAGCCTTTTTTTTTTG
GAGCTCTACTTAATTTCCGAGGAAAACCTCGGAAAAAAAAAACAGCT

with the large BamHI-SalI digested linear fragment of pBR322 to give plasmid pTerS21,
(l) digesting the plasmid pTerS21 with PstI and BamHI to give PstI-BamHI digested fragment (3 kbp),
(m) digesting the plasmid pLS-T2 with PstI and BamHI to give PstI-BamHI digested fragment (1.3 kbp),
(n) ligating the PstI-BamHI digested fragment (1.3 kbp) with the above PstI-BamHI digested fragment (3 kbp) to give plasmid pLHSdMmTer which is composed of PstI-Hind III digestible fragments (3.3 kbp and 1.0 kbp),
(o) digesting the plasmid pLHSdMmTer with SalI and PvuII to give SalI-PvuII digested fragment (2.9 kbp),
(p) treating the SalI-PvuII digested fragment (2.9 kbp) with DNA polymerase (large fragment) and dNTPs (dATP, dCTP, dGTP and TTP) to give a linear fragment having two flush end, and
(q) self-ligating the linear fragment to give plasmid pLS-D1.

**2.** A process for preparing the plasmid pLS-D2 comprising the following steps:
(a) digesting the plasmid pLS-D1 of claim 1 with BamHI to give BamHI digested pLS-D1 fragment,
(b) dephosphorlating the BamHI digested pLS-D1 fragment with CIP,
(c) isolating plasmid pLHSdMmtrp from E. coli F-6 FERM BP-729,
(d) digesting the plasmid pLHSdMmtrp with HpaI and PstI to give HpaI-PstI digested fragment (3.7 kbp),
(e) digesting partially the fragment (3.7 kbp) with HincII to give the HpaI-HincII digested fragment (732 bp),
(f) digesting plasmid pUC9 with HincII,
(g) dephosphorylating the resultant HincII digested fragment with calf intestinal alkaline phosphatase (CIP),
(h) ligating the HincII digested fragment of pUC9 with the above HpaI-HincII digested fragment (732 bp) to give plasmid pUC-SS1,
(i) digesting plasmid pUC-SS1 with BamHI to give BamHI digested pUC-SS1 fragment (464 bp), and
(j) ligating the BamHI digested pUC-ssl fragment (464 bp) with the dephosphorylated and BamHI digested pLS-D1 fragment to give plasmid pLS-D2.

**3.** A process for preparing the plasmid pLS-DD1 comprising the following steps:
(a) digesting the plasmid pLS-D1 of claim 1 with PstI to give PstI digested pLS-D1 fragment,
(b) dephosphorylating the PstI digested pLS-D1 fragment,
(c) digesting the plasmid pLS-D2 of claim 2 with PstI to give Pst I digested pLS-D2 fragment, and
(d) ligating the PstI digested pLS-D2 fragment with the dephosphorylated and PstI digested pLS-D1 fragment to give plasmid pLS-DD1

**4.** A process for transforming E. coli with the plasmid pLS-D1 obtained in claim 1.

**5.** A process for transforming E. coli with the plasmid pLS-D2 obtained in claim 2.

**6.** A process for transforming E. coli with the plasmid pLS-DD1 obtained in claim 3.

**7.** A process for Preparing IGF-I fused with a protective peptide by culturing a E. coli transformed with the expression plasmid pLS-D2 obtained in claim 1.

**8.** A process for preparing IGF-I fused with a protective peptide by culturing a E. coli transformed with the expression plasmid pLS-D2 obtained in claim 2.

**9.** A process for preparing IGF-I fused with a protective peptide by culturing a E. coli transformed with the expression plasmid pLS-DD1 obtained in claim 3.

**Revendications**

**Revendications pour les Etats contractant suivants : BE, CH, DE, FR, GB, IT, LI, LU, NL, SE**

1. Plasmide pLS-D1 que l'on peut obtenir par:
   (a) isolement du plasmide pLHSdMmtrp à partir de E. coli F-6 FERM BP-729,
   (b) digestion du plasmide pLHSdMmtrp par HpaI et PstI pour donner un fragment digéré HpaI-PstI (3,7 kpb),
   (c) digestion partielle du fragment (3,7 kpb) par HincII pour donner le fragment digéré HpaI-HincII (732 pb),
   (d) digestion du plasmide pUC9 par HincII,
   (e) déphosphorylation du fragment digéré HincII obtenu avec la phosphatase alcaline intestinale de veau (CIP),
   (f) soudure du fragment digéré par HincII de pUC9 par le fragment digéré HpaI-HincII (732 pb) pour donner le plasmide pUC-SS1,
   (g) digestion du plasmide pLHSdMmtrp par BamHI pour donner un fragment digéré par BamHI (4,5 kpb)
   (h) déphosphorylation du fragment (4,5 kpb) par CIP,
   (i) digestion du plasmide pUC-SS1 par BamHI pour donner un fragment digéré par BamHI (464 pb),
   (j) soudure du fragment digéré par BamHI (464 pb) avec le fragment déphosphorylé ci-dessus (4,5 kpb) pour donner le plasmide pLS-T2 qui est composé de deux fragments digestibles Pst1-PvuII (1,5 kpb et 3,4 kpb),
   (k) soudure du gène terminateur S de la séquence d'ADN suivante:

   GATCCTCGAGATCAATTAAAGGCTCCTTTTGGAGCCTTTTTTTTG
   GAGCTCTACTTAATTTCCGAGGAAAACCTCGGAAAAAAAAAACAGCT

   avec le grand fragment linéaire digéré de pBR322 pour donner le plasmide pTerS21,
   (l) digestion du plasmide pTerS21 par PstI et BamHI pour donner le fragment digéré PstI-BamHI (3 kpb),
   (m) digestion du plasmide pLS-T2 par PstI et BamHI pour donner le fragment digéré PstI-BamHI (1,3 kpb),
   (n) soudure du fragment digéré PstI-BamHI (1,3 kpb) avec le fragment digéré PstI-BamHI ci-dessus (3 kpb) pour donner le plasmide pLHSdMmTer qui est composé de fragments digestibles PstI-Hind III (3,3 kpb et 1,0 kpb)
   (o) digestion du plasmide pLHSdMmTer par SalI et PvuII pour donner le fragment digéré SalI-PvuII (2,9 kpb)
   (p) traitement du fragment digéré SalI-PvuII (2,9 kpb) par de l'ADN polymérase (grand fragment) et par des dNTP (dATP, dCTP, dGTP, et TTP) pour donner un fragment linéaire possédant deux extrémités droites, et
   (q) autosoudure du fragment linéaire pour donner un plasmide pLS-D1.

2. Plasmide pLS-D2 pouvant être obtenu par :
   (a) digestion du plasmide pLS-D1 de la revendication 1 par BamHI pour donner le fragment pLS-D1 digéré par BamHI,
   (b) déphosphorylation du fragment pLS-D1 digéré par BamHI par CIP,
   (c) isolement du plasmide pLHSdMmtrp à partir de E. coli F-6 FERM BP-729,
   (d) digestion du plasmide pLHSdMmtrp par HpaI et PstI pour donner le fragment digéré HpaI-PstI (3,7 kpb),
   (e) digestion partielle du fragment (3,7 kpb) par HincII pour donner le fragment digéré HpaI-HincII (732 pb),
   (f) digestion du plasmide pUC9 par HincII,
   (g) déphosphorylation du fragment digéré HincII obtenu par la phosphatase alcaline intestinale de veau (CIP),
   (h) soudure du fragment digéré HincII de pUC9 avec le fragment digéré HpaI-HincII (732 pb) pour donner le plasmide pUC-SS1,

(i) digestion du plasmide pUC-SS1 par BamHI pour donner le fragment pUC-SS1 digéré par BamHI (464 pb), et

(j) soudure du fragment pUC-SS1 digéré par BamHI (464 pb) avec le fragment pLS-D1 déphosphorylé et digéré par BamHI pour donner le plamide pLS-D2.

**3.** Plasmide pLS-DD1 pouvant être obtenu par :

(a) digestion du plasmide pLS-D1 de la revendication 1 par PstI pour donner le fragment pLS-D1 digéré par PstI,

(b) déphosphorylation du fragment pLS-D1 digéré par PstI

(c) digestion du plasmide pLS-D2 de la revendication 2 par PstI pour donner le fragment pLS-D2 digéré par PstI, et

(d) soudure du fragment pLS-D2 digéré par PstI avec le fragment pLS-D1 déphosphorylé et digéré par PstI pour donner le plasmide pLS-DD1

**4.** E. coli transformé par le plasmide pLS-D1 obtenu dans la revendication 1.

**5.** E. coli transformé par le plasmide pLS-D2 obtenu dans la revendication 2.

**6.** E. coli par transformé par le plasmide pLS-DD1 obtenu dans la revendication 3.

**7.** Procédé de préparation d'un IGF-I fusionné avec un peptide protecteur en cultivant un E. coli transformé par le plasmide d'expression pLS-D1 obtenu dans la revendication 1.

**8.** Procédé de préparation d'un IGF-I fusionné avec un peptide protecteur en cultivant un E. coli transformé par le plasmide d'expression pLS-D2 obtenu dans la revendication 2.

**9.** Procédé de préparation d'un IGF-I fusionné avec un peptide protecteur en cultivant un E. coli transformé par le plasmide d'expression pLS-DD1 obtenu dans la revendication 3.

**Revendications pour les Etats contractants suivants : AT, ES, GR**

**1.** Procédé de préparation du plasmide pLS-D1 comprenant les étapes suivantes :

(a) isolement du plasmide pLHsdMmtrp à partir de E. coli F-6 FERM BP-729,

(b) digestion du plasmide pLHSdMmtrp par HpaI et PstI pour donner un fragment digéré HpaI-PstI (3,7 kpb),

(c) digestion partielle du fragment (3,7 kpb) par HincII pour donner le fragment digéré HpaI-HincII (732 pb),

(d) digestion du plasmide pUC9 par HincII,

(e) déphosphorylation du fragment digéré HincII obtenu avec la phosphatase alcaline intestinale de veau (CIP),

(f) soudure du fragment digéré par HincII de pUC9 avec le fragment digéré HpaI-HincII (732 pb) pour donner le plasmide pUC-SS1,

(g) digestion du plasmide pLHSdMmtrp par BamHI pour donner un fragment digéré par BamHI (4,5 kpb)

(h) déphosphorylation du fragment (4,5 kpb) par CIP,

(i) digestion du plasmide pUc-SS1 par BamHI pour donner un fragment digéré par BamHI (464 pb),

(j) soudure du fragment digéré par BamHI (464 pb) avec le fragment déphosphorylé ci-dessus (4,5 kpb) pour donner le plasmide pLS-T2 qui est composé de deux fragments digestibles Pst1-PvuII (1,5 kpb et 3,4 kpb),

(k) soudure du gène terminateur S de la séquence d'ADN suivante:

GATCCTCGAGATCAATTAAAGGCTCCTTTTGGAGCCTTTTTTTTG

GAGCTCTACTTAATTTCCGAGGAAAACCTCGGAAAAAAAAAACAGCT

avec le grand fragment linéaire digéré de pBR322 pour donner le plasmide pTerS21,

(l) digestion du plasmide pTerS21 par PstI et BamHI pour donner le fragment digéré PstI-BamHI (3 kpb),
(m) digestion du plasmide pLS-T2 par PstI et BamHI pour donner le fragment digéré PstI-BamHI (1,3 kpb),
(n) soudure du fragment digéré PstI-BamHI (1,3 kpb) avec le fragment digéré PstI-BamHI ci-dessus (3 kpb) pour donner le plasmide pLHSdMmTer qui est composé de fragments digestibles PstI-Hind III (3,3 kpb et 1,0 kpb)
(o) digestion du plasmide pLHSdMmTer par SalI et PvuII pour donner le fragment digéré SalI-PvuII (2,9 kpb)
(p) traitement du fragment digéré SalI-PvuII (2,9 kpb) par de l'ADN polymérase (grand fragment) et par des dNTP (dATP, dCTP, dGTP, et TTP) pour donner un fragment linéaire possédant deux extrémités droites, et
(q) autosoudure du fragment linéaire pour donner un plasmide pLS-D1.

**2.** Procédé de préparation du plasmide pLS-D2 comprenant les étapes suivantes :
(a) digestion du plasmide pLS-D1 de la revendication 1 par BamHI pour donner le fragment pLS-D1 digéré par BamHI,
(b) déphosphorylation du fragment pLS-D1 digéré par BamHI par CIP,
(c) isolement du plasmide pLHSdMmtrp à partir de E. coli F-6 FERM BP-729,
(d) digestion du plasmide pLHSdMmtrp par HpaI et PstI pour donner le fragment digéré HpaI-PstI (3,7 kpb),
(e) digestion partielle du fragment (3,7 kpb) par HincII pour donner le fragment digéré HpaI-HincII (732 pb),
(f) digestion du plasmide pUC9 par HincII,
(g) déphosphorylation du fragment digéré HincII obtenu par la phosphatase alcaline intestinale de veau (CIP),
(h) soudure du fragment digéré HincII de pUC9 avec le fragment digéré HpaI-HincII (732 pb) pour donner le plasmide pUC-SS1,
(i) digestion du plasmide pUC-SS1 par BamHI pour donner le fragment pUC-SS1 digéré par BamHI (464 pb), et
(j) soudure du fragment pUC-SS1 digéré BamHI (464 pb) avec le fragment pLS-D1 déphosphorylé et digéré par BamHI pour donner le plasmide pLS-D2.

**3.** Procédé de préparation du plasmide pLS-DD1 comprenant les deux étapes suivantes :
(a) digestion du plasmide pLS-D1 de la revendication 1 par PstI pour donner le fragment pLS-D1 digéré par PstI,
(b) déphosphorylation du fragment pLS-D1 digéré par PstI
(c) digestion du plasmide pLS-D2 de la revendication 2 par PstI pour donner le fragment pLS-D2 digéré par PstI, et
(d) soudure du fragment pLS-D2 digéré par PstI avec le fragment pLS-D1 déphosphorylé et digéré par PstI pour donner le plasmide pLS-DD1

**4.** Procédé de transformation de E. coli par le plasmide pLS-D1 obtenu dans la revendication 1.

**5.** Procédé de transformation de E. coli par le plasmide pLS-D2 obtenu dans la revendication 2.

**6.** Procédé de transformation de E. coli par le plasmide pLS-DD1 obtenu dans la revendication 3.

**7.** Procédé de préparation d'un IGF-I fusionné avec un peptide protecteur en cultivant un E. coli transformé par le plasmide d'expression pLS-D1 obtenu dans la revendication 1.

**8.** Procédé de préparation d'un IGF-I fusionné avec un peptide protecteur en cultivant un E. coli transformé par le plasmide d'expression pLS-D2 obtenu dans la revendication 2.

**9.** Procédé de préparation d'un IGF-I fusionné avec un peptide protecteur en cultivant un E. coli transformé par le plasmide d'expression pLS-DD1 obtenu dans la revendication 3.

**Patentansprüche**

**Patentansprüche für folgende Vertragsstaaten : BE, CH, DE, SE FR, GB, IT, LI, LU, NL, SE**

1. Plasmid pLS-D1, das erhältlich ist durch
   (a) Isolieren des Plasmids pLHSdMmtrp aus E. Coli F-6 FERM BP 729,
   (b) Digestieren des Plasmids pLHSdMmtrp mit HpaI und PstI zur Herstellung des HpaI-PstI-digestierten Fragments (3,7 kbp),
   (c) partielles Digestieren des Fragments (3,7 kbp) mit HincII zur Herstellung des HpaI-HincII-digestierten Fragments (732 bp),
   (d) Digestieren des Plasmids pUC9 mit HincII,
   (e) Dephosphorylieren des resultierenden HincII-digestierten Fragments mit alkalischer Kalbsintestinal-Phosphatase (CIP),
   (f) Ligieren des HincII-digestierten Fragments von pUC9 mit dem obengenannten HpaI-HincII-digestierten Fragment (732 bp) zur Herstellung des Plasmids pUC-SS1,
   (g) Digestieren des Plasmids pLHSdMmtrp mit BamHI zur Herstellung des BamHI-digestierten Fragments (4,5 kbp),
   (h) Dephosphorylieren des Fragments (4,5 kbp) mit CIP,
   (i) Digestieren des Plasmids pUC-SS1 mit BamHI zur Herstellung des BamHI-digestierten Fragments (464 bp),
   (j) Ligieren des BamHI-digestierten Fragments (464 bp) mit dem obengenannten dephosphorylierten Fragment (4,5 kbp) zur Herstellung des Plasmids pLS-T2, das besteht aus zwei Pst1-PvuII-digestierbaren Fragmenten (1,5 kbp und 3,4 kbp),
   (k) Ligieren des Terminator S-Gens mit der nachfolgenden DNA-Sequenz:

   GATCCTCGAGATCAATTAAAGGCTCCTTTTGGAGCCTTTTTTTTTTG
   GAGCTCTACTTAATTTCCGAGGAAAACCTCGGAAAAAAAAAACAGCT

   mit dem großen linearen BamHI-SalI-digestieren Fragment pBR322 zur Herstellung des Plasmids pTerS21,
   (l) Digestieren des Plasmids PTerS21 mit PstI und BamHI zur Herstellung des PstI-BamHI-digestierten Fragments (3 kbp),
   (m) Digestieren des Plasmids pLS-T2 mit PstI und BamHI zur Herstellung des PstI-BamHI-digestierten Fragments (1,3 kbp),
   (n) Ligieren des PstI-BamHI-digestierten Fragments (1,3 kbp) mit dem obengenannten PstI-BamHI-digestierten Fragment (3 kbp) zur Herstellung des plasmids pLSHdMmTer, das besteht aus den PstI-HindIII-digestierbaren Fragmenten (3,3 kbp und 1,0 kbp),
   (o) Digestieren des Plasmids pLHSdMmTer mit Sa1l und PvuII zur Herstellung des Salz-PvuII-digestierten Fragments (2,9 kbp),
   (p) Behandeln des SalI-PvuII-digestierten Fragments (2.9 kbp) mit DNA-Polymerase (großes Fragment) und dNTPs (dATP, dCTP, dGTP und TTP) zur Herstellung eines linearen Fragments mit zwei glatten Enden und
   (q) Selbst-Ligieren des linearen Fragments zur Herstellung des Plasmids pLS-D1.

2. Plasmid pLS-D2, das erhältlich ist durch
   (a) Digestieren des Plasmids pLS-D1 des Anspruchs 1 mit BamHI zur Herstellung des BamHI-digestieren pLS-D1-Fragments,
   (b) Dephosphorylieren des BamHI-digestierten pLS-D1-Fragments mit CIP,
   (c) Isolieren des Plasmids pLHSdMmtrp aus E. Coli F-6 FERM BP-729,
   (d) Digestieren des Plasmids pLHSdMmtrp mit HpaI und PstI zur Herstellung des HpaI-PstI-digestierten Fragments (3,7 kbp),
   (e) partielles Digestieren des Fragments (3,7 kbp) mit HincII zur Herstellung des HpaI-HincII-digestierten Fragments (732 bp),
   (f) Digestieren des Plasmids pUC9 mit HincII,
   (g) Dephosphorylieren des resultierenden HincII-digestierten Fragments mit alkalischer Kalbsintestinal-Phosphatase (CIP),
   (h) Ligieren des HincII-digestierten Fragments von pUC9 mit dem obengenannten HpaI-HincII-digestierten Fragment (732bp) zur Herstellung des Plasmids pUC-SS1,

(i) Digestieren des Plasmids pUC-SS1 mit BamHI zur Herstellung des BamHI-digestierten pUC-SS1-Fragments (464 bp), und
(j) Ligieren des BamHI-digestierten pUC-SS1-Fragments (464 bp) mit dem dephosphorylierten und BamHI-digestierten pLS-D1-Fragmentszur Herstellung des plasmids pLS-D2.

3. Plasmid pLS-DD1, das erhältlich ist durch
(a) Digestieren des Plasmids pLS-D1 des Anspruchs 1 mit PstI zur Herstellung des PstI-digestierten pLS-D1-Fragments,
(b) Dephosphorylieren des PstI-digestierten pLS-D1-Fragments,
(c) Digestieren des Plasmids pLS-D2 des Anspruchs 2 mit PstI zur Herstellung des PstI-digestierten pLS-D2-Fragments und
(d) Ligieren des pstI-digestierten pLS-D2-Fragments mit dem dephosphorylierten und PstI-digestierten PLS-D1-Fragment zur Herstellung des Plasmids pLS-DD1.

4. E. Coli, transformiert mit dem Plasmid pLS-D1 des Anspruchs 1.

5. E. Coli, transformiert mit dem Plasmid pLS-D2 des Anspruchs 2.

6. E. Coli, transformiert mit dem Plasmid pLS-DD1 des Anspruchs 3.

7. Verfahren zur Herstellung von IGF-I,der mit einem Schutz-Peptid fusioniert ist, durch Kultivieren eines E. Coli, das mit dem Expressions-Plasmid pLS-D1 des Anspruchs 1 transformiert worden ist.

8. Verfahren zur Herstellung von IGF-I, der mit einem Schutz-Peptid fusioniert worden ist, durch Kultivieren eines E. Coli, das mit dem Expressions-Plasmid pLS-D2 des Anspruchs 2 transformiert worden ist.

9. Verfahren zur Herstellung von IGF-I, der mit einem Schutz-Peptid fusioniert ist, durch Kultivieren eines E. Coli, das mit dem Expressions-Plasmid pLS-DD1 des Anspruchs 3 trans-formiert worden ist.

**Patentansprüche für folgende Vertragsstaaten : AT, ES, GR**

1. Verfahren zur Herstellung des Plasmid pLS-D1, das die folgenden Stufen umfaßt:
(a) Isolieren des Plasmids pLHSdMmtrp aus E. Coli F-6 FERM BP-729,
(b) Digestieren des Plasmids pLHSdMmtrp mit HpaI und PstI zur Herstellung des HpaI-PstI-digestierten Fragments (3,7 kbp),
(c) partielles Digestieren des Fragments (3,7 kbp) mit HincII zur Herstellung des HpaI-HincII-digestierten Fragments (732 bp),
(d) Digestieren des Plasmids pUC9 mit HincII,
(e) Dephosphorylieren des resultierenden HincII-digestierten Fragments mit alkalischer Kalbsintestinalphosphatase (CIP),
(f) Ligieren des HincII-digestierten Fragments von pUC9 mit dem obengenannten HpaI-HincII-digestierten Fragment (732 bp) zur Herstellung des Plasmids pUC-SS1,
(g) Digestieren des Plasmids pLHSdMmtrp mit BamHI zur Herstellung des BamHI-digestierten Fragments (4,5 kbp),
(h) Dephosphorylieren des Fragments (4,5 kbp) mit CIP,
(i) Digestieren des Plasmids pUC-SS1 mit BamHI zur Herstellung des BamHI-digestierten Fragments (464 bp),
(j) Ligieren des BamHI-digestierten Fragments (464 bp) mit dem obengenannten dephosphorylierten Fragment (4,5 kbp) zur Herstellung des Plasmids pLS-T2, das besteht aus zwei Pst1-PvuII-digestierbaren Fragmenten (1,5 kbp und 3,4 kbp),
(k) Ligieren des Terminator S-Gens mit der folgenden DNA-Sequenz:

GATCCTCGAGATCAATTAAAGGCTCCTTTTGGAGCCTTTTTTTTTTG
GAGCTCTACTTAATTTCCGAGGAAAACCTCGGAAAAAAAAAACAGCT

mit dem großen BamHI-SalI-digestierten linearen Fragment von pBR322 zur Herstellung des Plas-

mids pTerS21,
(l) Digestieren des Plasmids pTerS21 mit PstI und BamHI zur Herstellung eines PstI-BamHI-digestierten Fragments (3 kbp),
(m) Digestieren des Plasmids pLS-T2 mit PstI und BamHI zur Herstellung eines PstI-BamHI-digestierten Fragments (1,3 kbp),
(n) Ligieren des PstI-BamHI-digestierten Fragments (1,3 kbp) mit dem obengenannten PstI-BamHI-digestierten Fragment (3 kbp) zur Herstellung des Plasmids pLHSdMmTer, das besteht aus PstI-HindIII-digestierbaren Fragmenten (3,3 kbp und 1,0 kbp),
(o) Digestieren des Plasmids pLHSdMmTer mit SalI und PvuII zur Herstellung eines SalI-PvuII-digestierten Fragments (2,9 kbp),
(p) Behandeln des SalI-PvuII-digestierten Fragments(2,9 kbp) mit DNA-Polymerase (großes Fragment) und dNTPs (dATP, dCTP, dGTP und TTP) zur Herstellung eines linearen Fragments mit zwei glatten Enden und
(q) Selbst-Ligieren des linearen Fragments zur Herstellung des Plasmids pLS-D1.

**2.** Verfahren zur Herstellung des Plasmids pLS-D2, das die folgenden Stufen umfaßt:
(a) Digestieren des Plasmids pLS-D1 des Anspruchs 1 mit BamHI-zur Herstellung eines BamHI-digestierten pLS-D1-Fragments,
(b) Dephosphorylieren des BamHI-digestierten pLS-D1-Fragments mit CIP,
(c) Isolieren des Plasmids pLHSdMmtrp aus E. coli F-6 FERM BP-729,
(d) Digestieren des Plasmids pLHSdMmtrp mit HpaI und PstI zur Herstellung eines HpaI-PstI-digestierten Fragments (3,7 kbp),
(e) partielles Digestieren des Fragments (3,7 kbp) mit HincII zur Herstellung eines HpaI-HincII-digestierten Fragments (732 bp),
(f) Digestieren des Plasmids pUC9 mit HincII,
(g) Dephosphorylieren des resultierenden HincII-digestierten Fragments mit alkalischer Kalbsintestinal-Phosphatase (CIP),
(h) Ligieren des HincII-digestierten Fragments von pUC9 mit dem obengenannten HpaI-HincII-digestierten Fragment (732 bp) zur Herstellung des Plasmids pUC-SS1,
(i) Digestieren des Plasmids pUC-SS1 mit BamHI zur Herstellung eines BamHI-digestierten pUC-SS1-Fragments (464 bp) und
(j) Ligieren des BamHI-digestierten pUC-SS1-Fragments (464 bp) mit dem dephosphorylierten und BamHI-digestierten pLS-D1-Fragment zur Herstellung des Plasmids pLS-D2.

**3.** Verfahren zur Herstellung des Plasmids pLS-DD1, das die folgenden Stufen umfaßt:
(a) Digestieren des Plasmids pLS-D1 des Anspruchs 1 mit PstI zur Herstellung eines PstI-digestierten pLS-D1-Fragments,
(b) Dephosphorylieren des PstI-digestierten pLS-D1-Fragments,
(c) Digestieren des Plasmids pLS-D2 des Anspruchs 2 mit PstI zur Herstellung eines PstI-digestierten pLS-D2-Fragments und
(d) Ligieren des PstI-digestierten pLS-D2-Fragments mit dem dephosphorylierten, PstI-digestierten pLS-D1-Fragment zur Herstellung des Plasmids pLS-DD1.

**4.** Verfahren zum Transformieren von E. Coli mit dem in Anspruch 1 erhaltenen PlasmidpLs-D1.

**5.** Verfahren zum Transformieren von E. Coli mit dem in Anspruch 2 erhaltenen Plasmid pLS-D2.

**6.** Verfahren zum Transformieren von E. Coli mit dem in Anspruch 3 erhaltenen Plasmid pLS-DD1.

**7.** Verfahren zur Herstellung von IGF-I, der mit einem Schutz-Peptid fusioniert ist, durch Kultivieren eines E. Coli, das mit dem in Anspruch 1 erhaltenen Expressionsplasmid pLS-D1 transformiert worden ist.

**8.** Verfahren zur Herstellung von IGF-I, der mit einem Schutz-Peptid fusioniert ist, durch Kultivieren eines E. Coli, das mit dem in Anspruch 2 erhaltenen Expressionsplasmid pLS-D2 transformiert worden ist.

**9.** Verfahren zur Herstellung von IGF-I, der mit einem Schutz-Peptid fusioniert ist, durch Kultivieren eines E. Coli, das mit dem in Anspruch 3 erhaltenen Expressionsplasmid pLS-DD1 transformiert worden ist.

Fig. 1 Preparation of IGF-I gene (224bp)

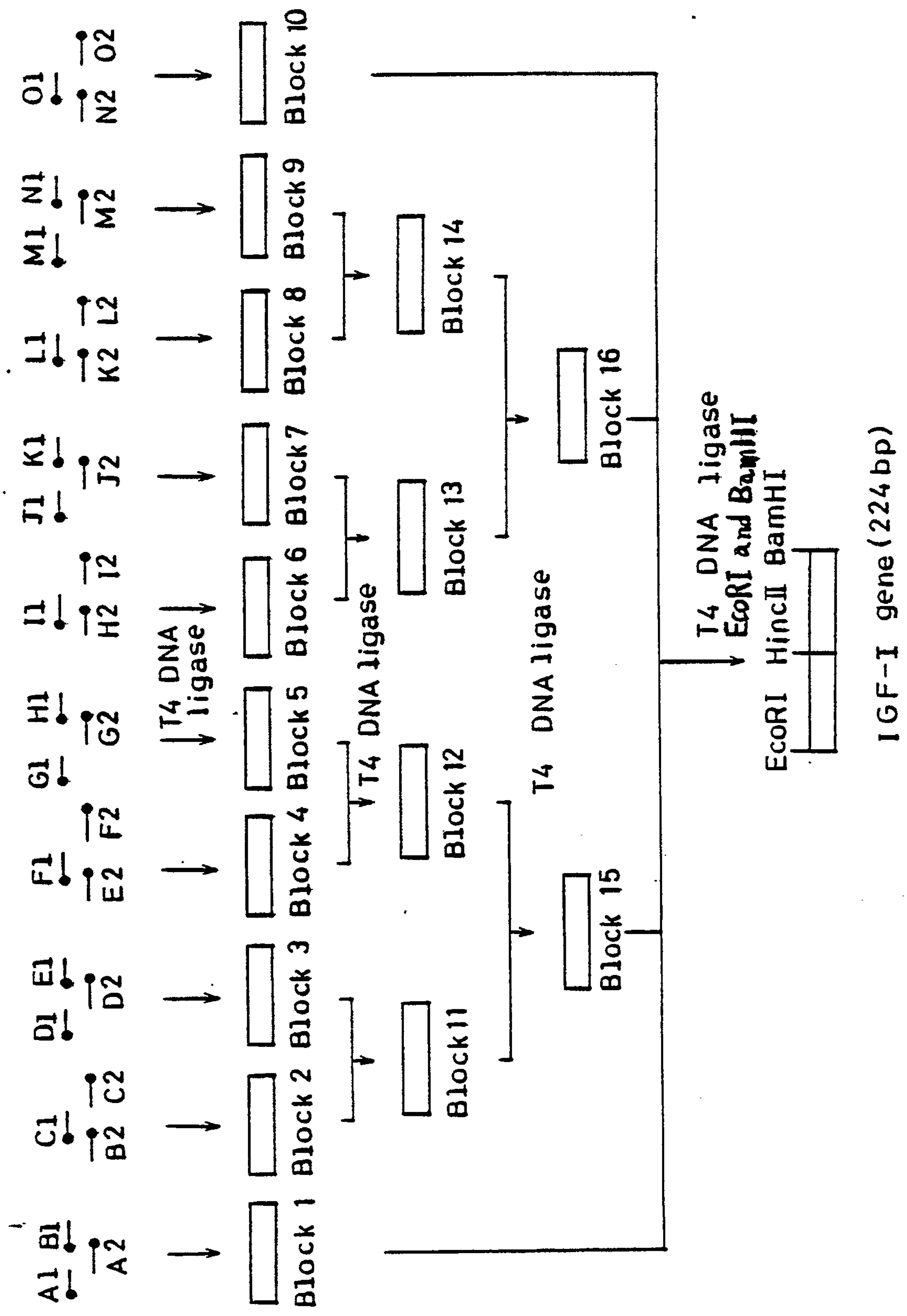

Fig.2 Construction of plasmid pSdM1

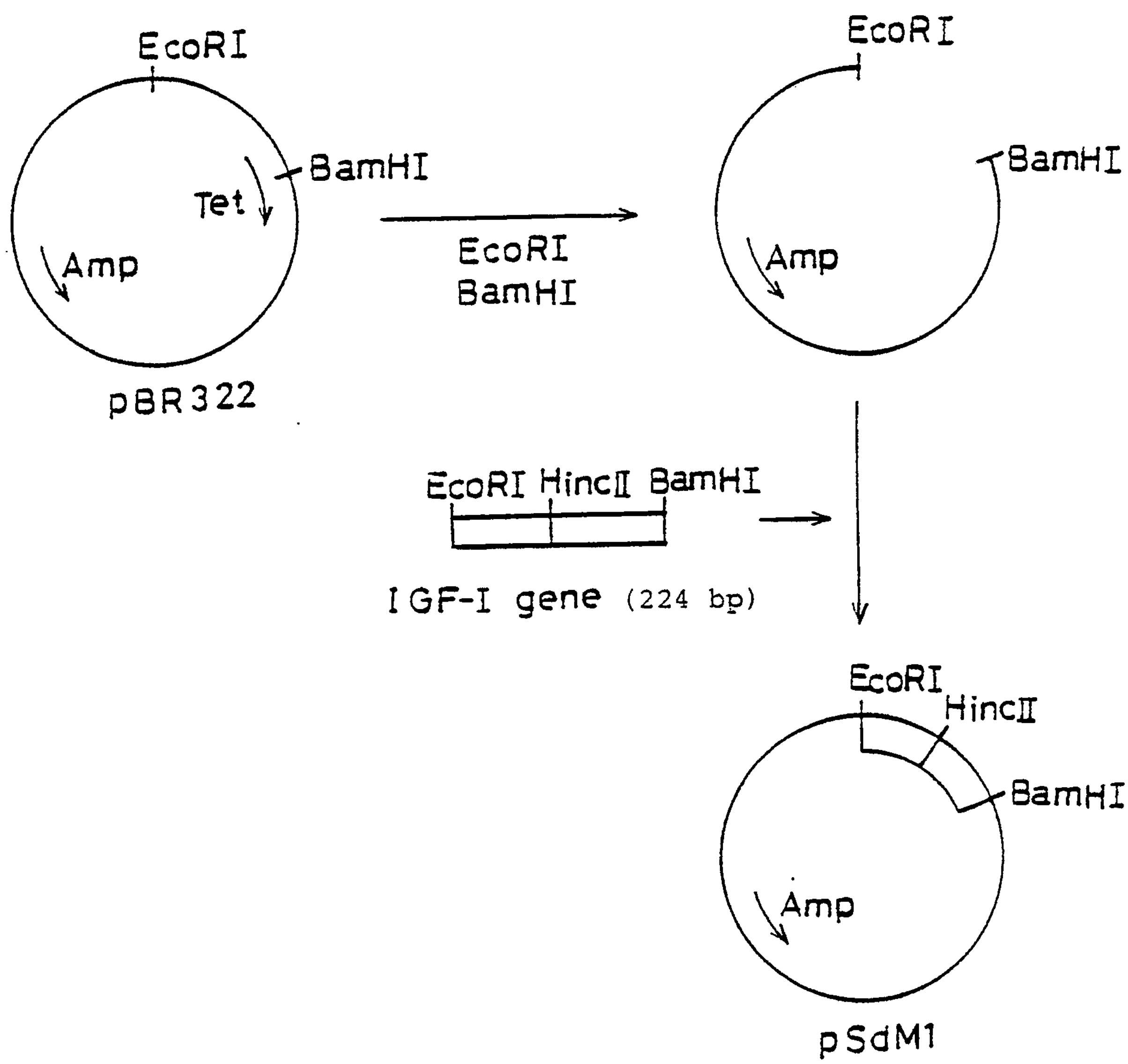

Fig. 3 Construction of synthetic trp promoter II gene

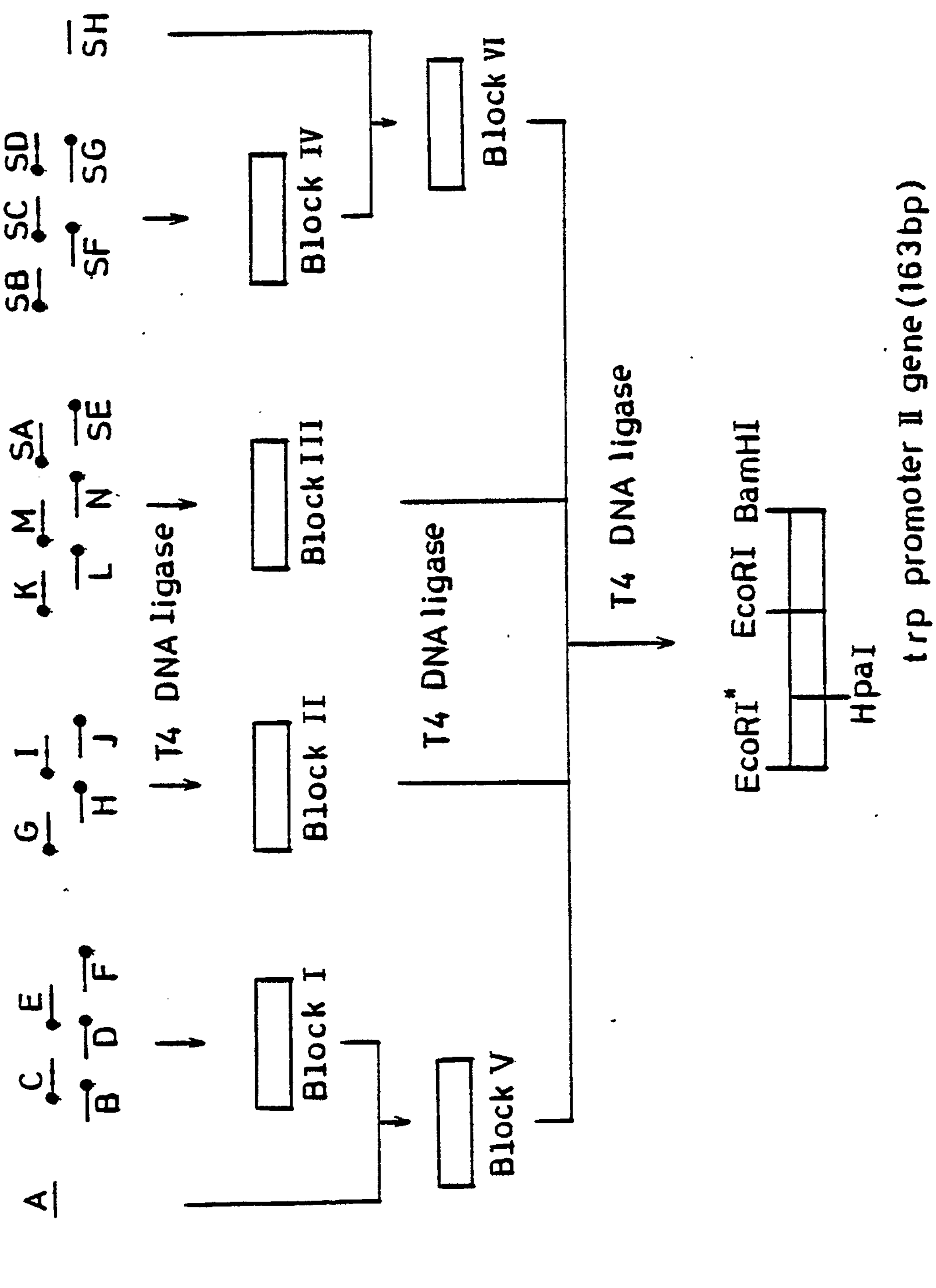

Fig.4 Construction of plasmid pTrpEB7

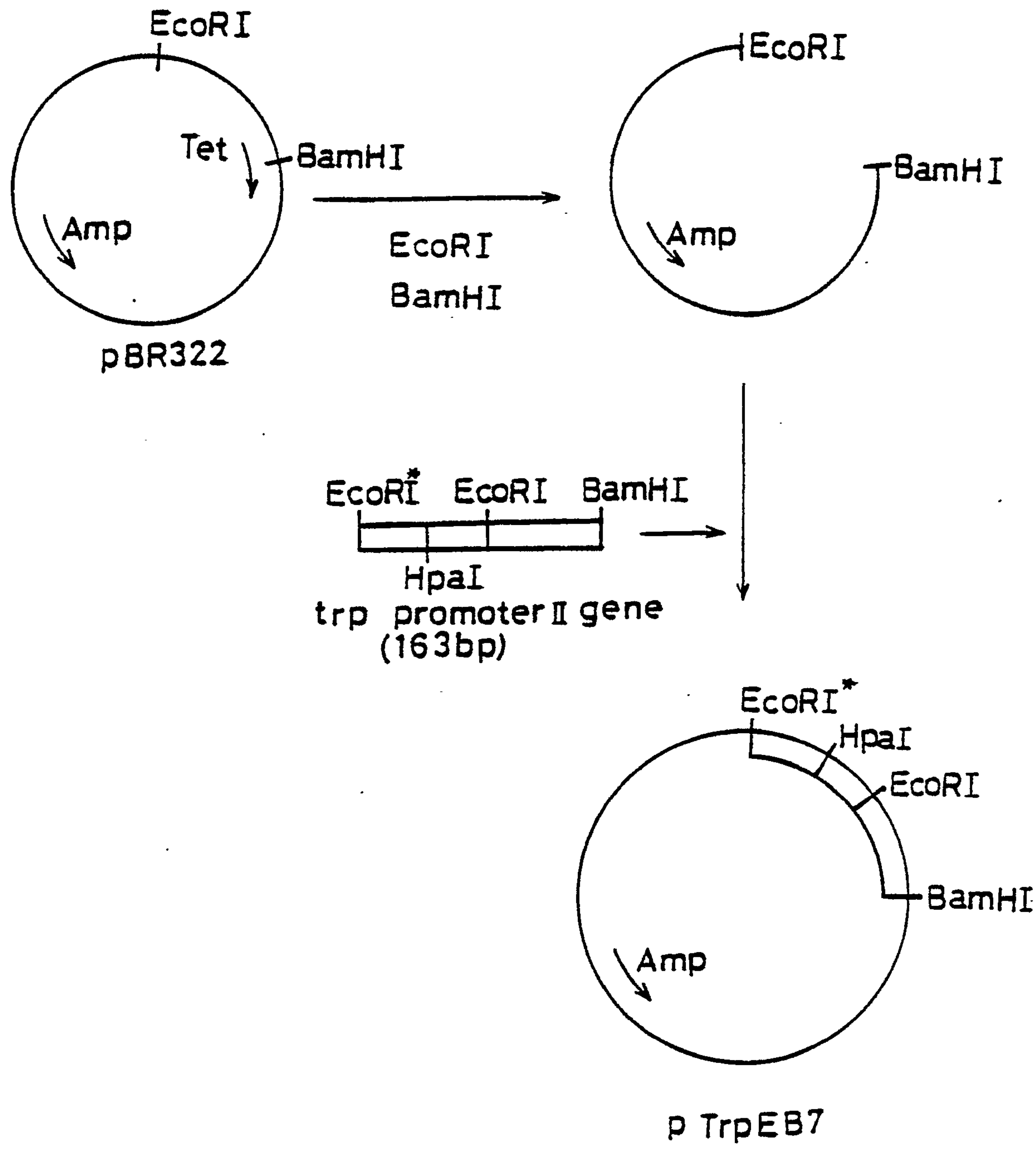

Fig.5 Construction of protein peptide LH gene (236bp)

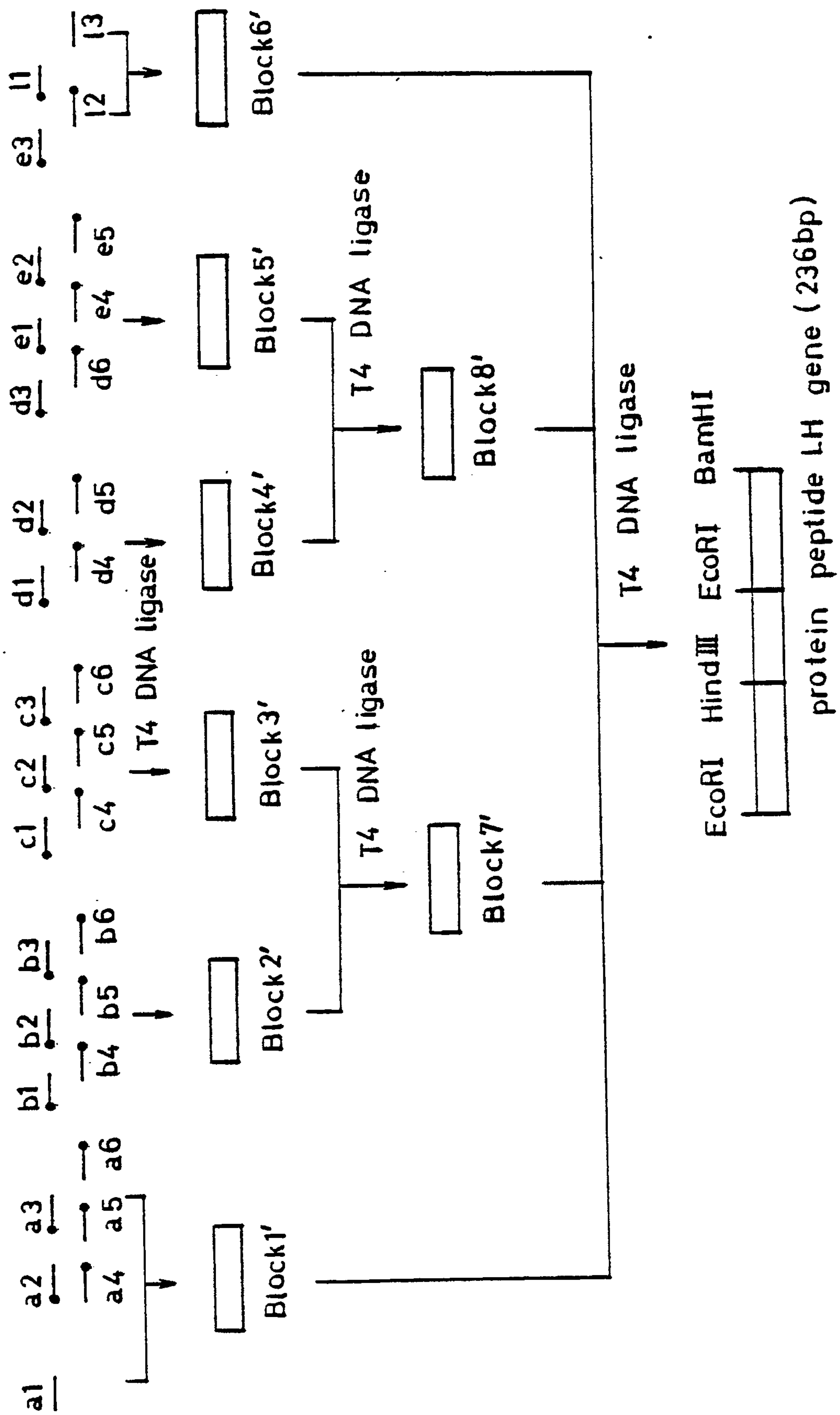

Fig.6 Construction of plasmid pLH107

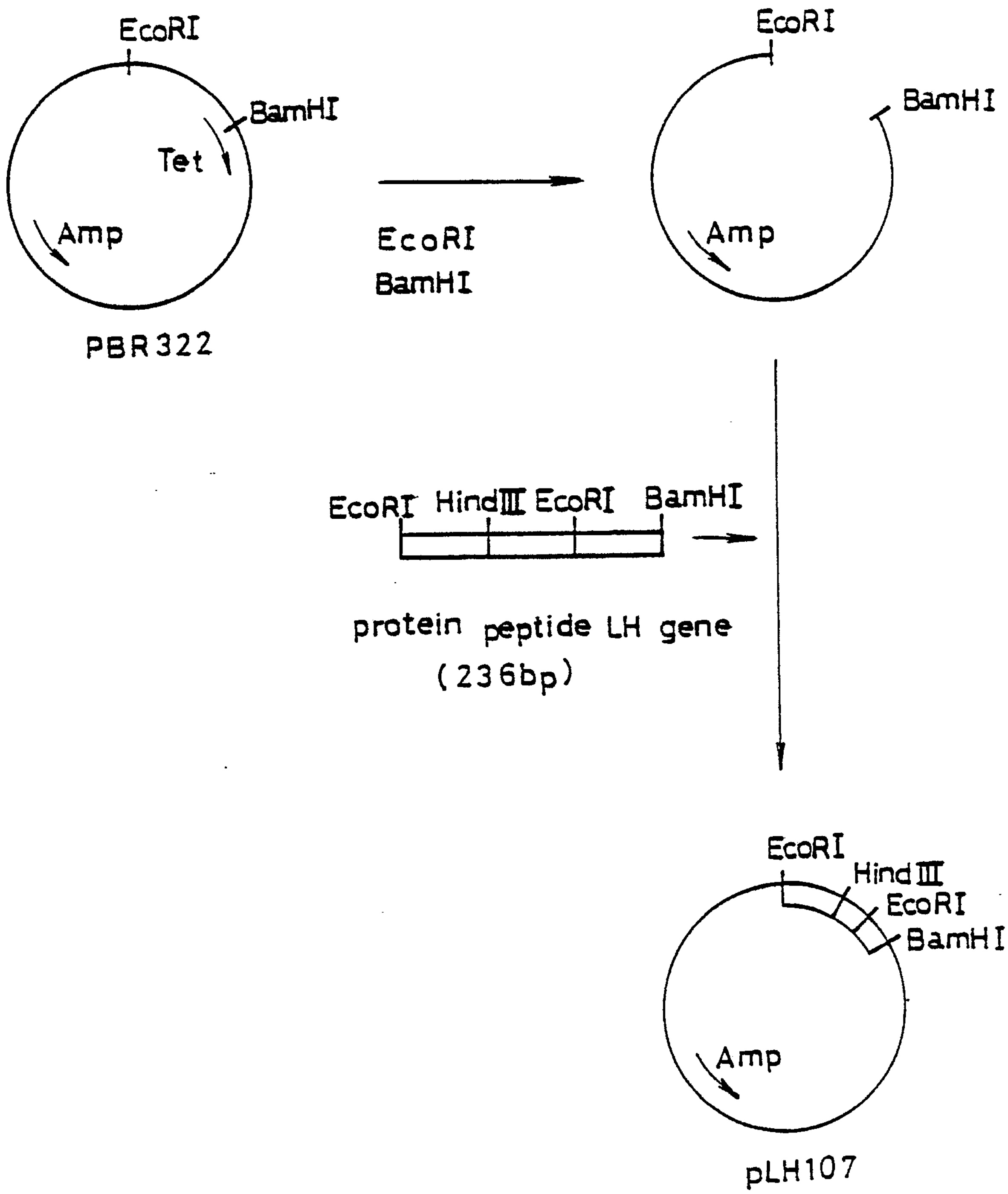

Fig.7 Construction of plasmid pLHtrp

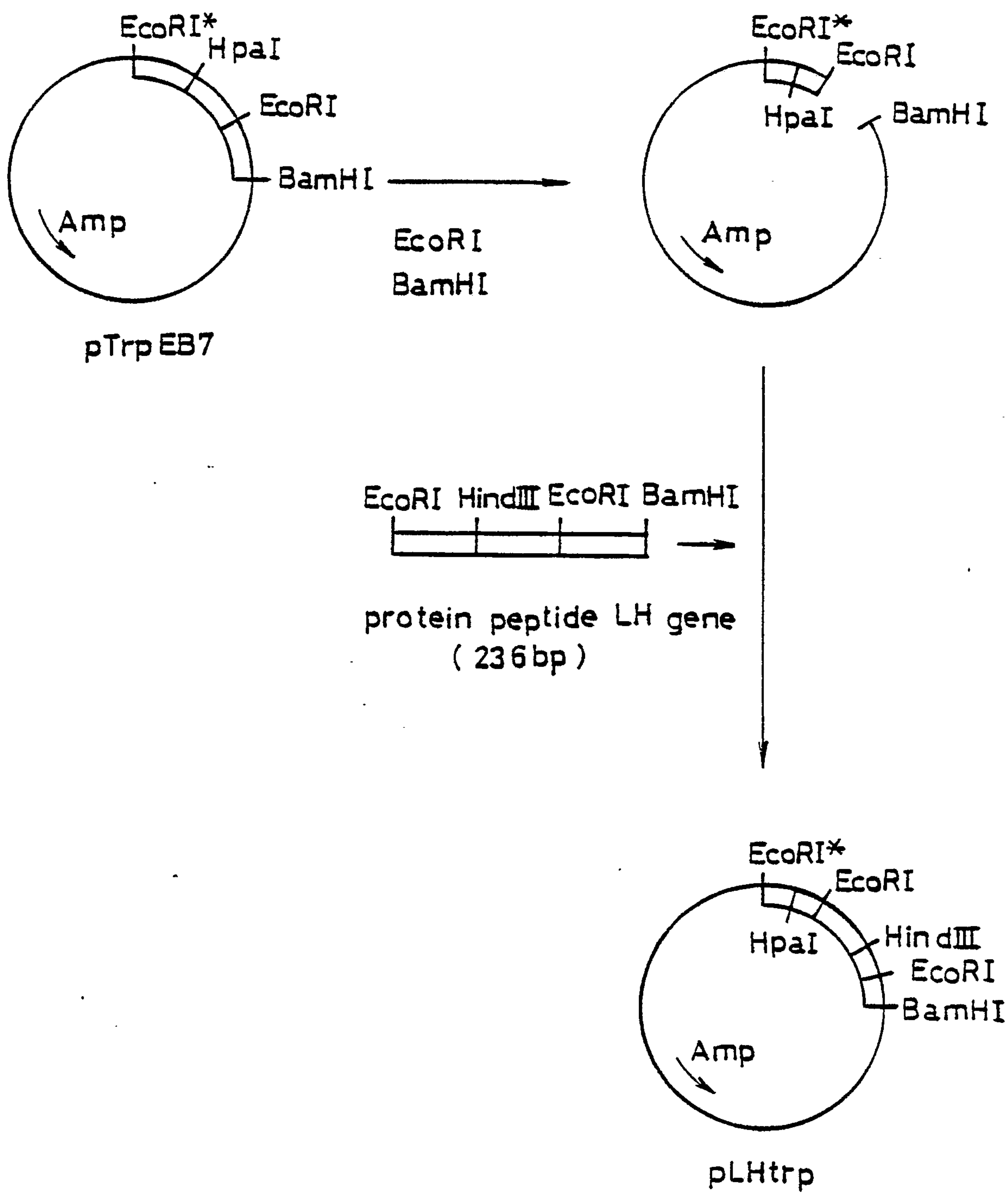

Fig. 8 Construction of plasmid pLHSdMmtrp

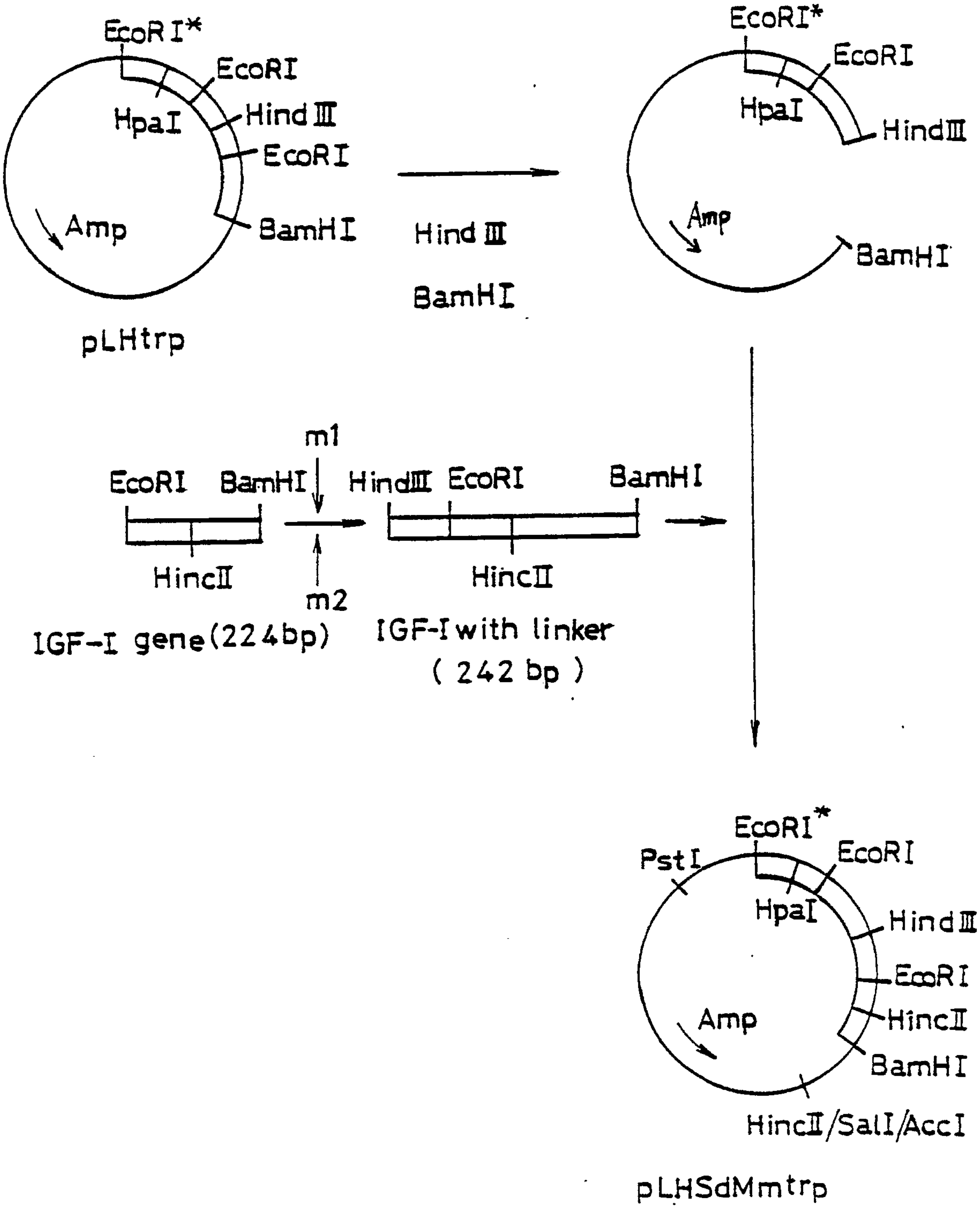

Fig.9 Construction of plasmid pUC-SS1(part1)

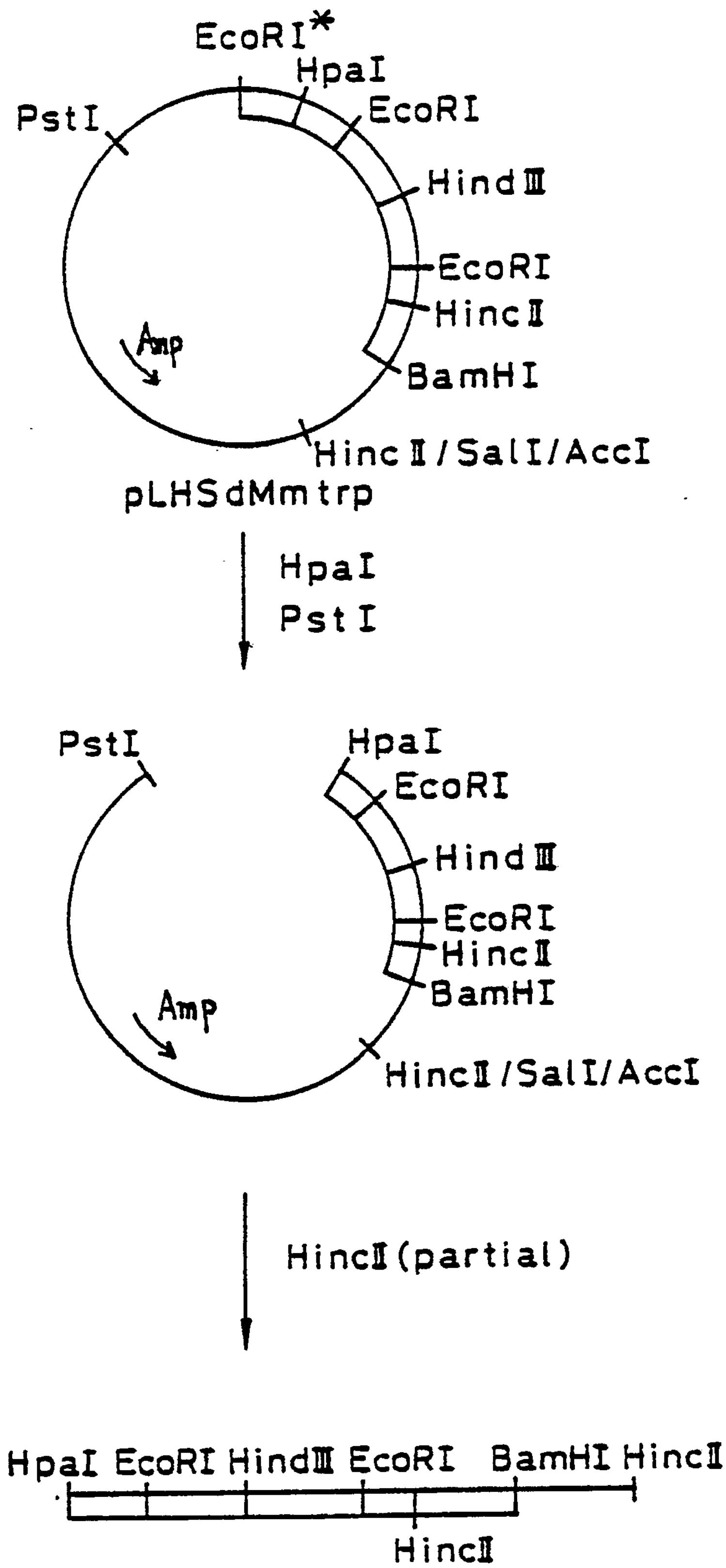


fused IGF-I gene (732bp)

Fig.10 Construction of plasmid pUC-SS1 (part2)

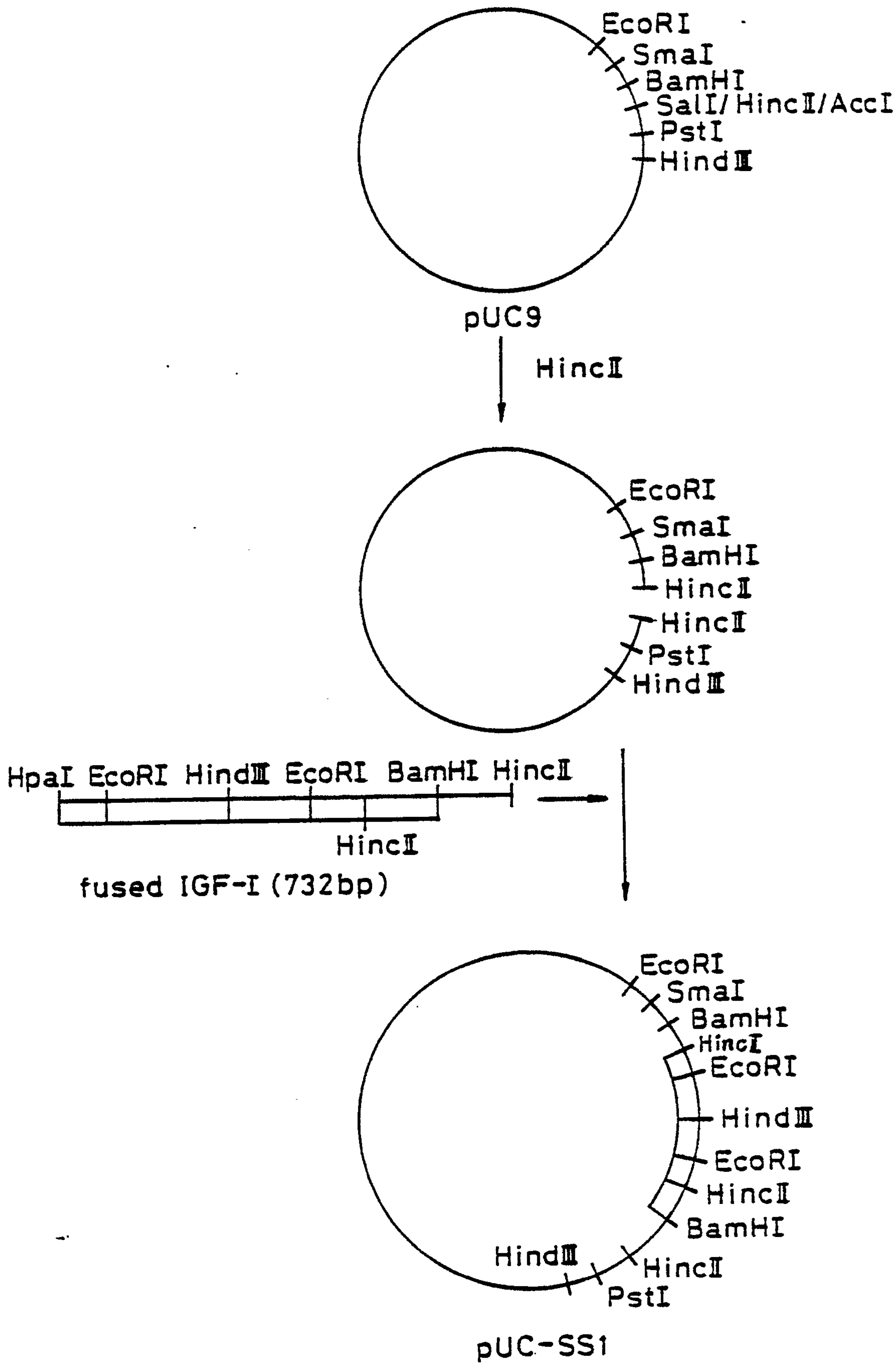

Fig.11 Construction of plasmid pLS-T2 and pLS-T3 (part1)

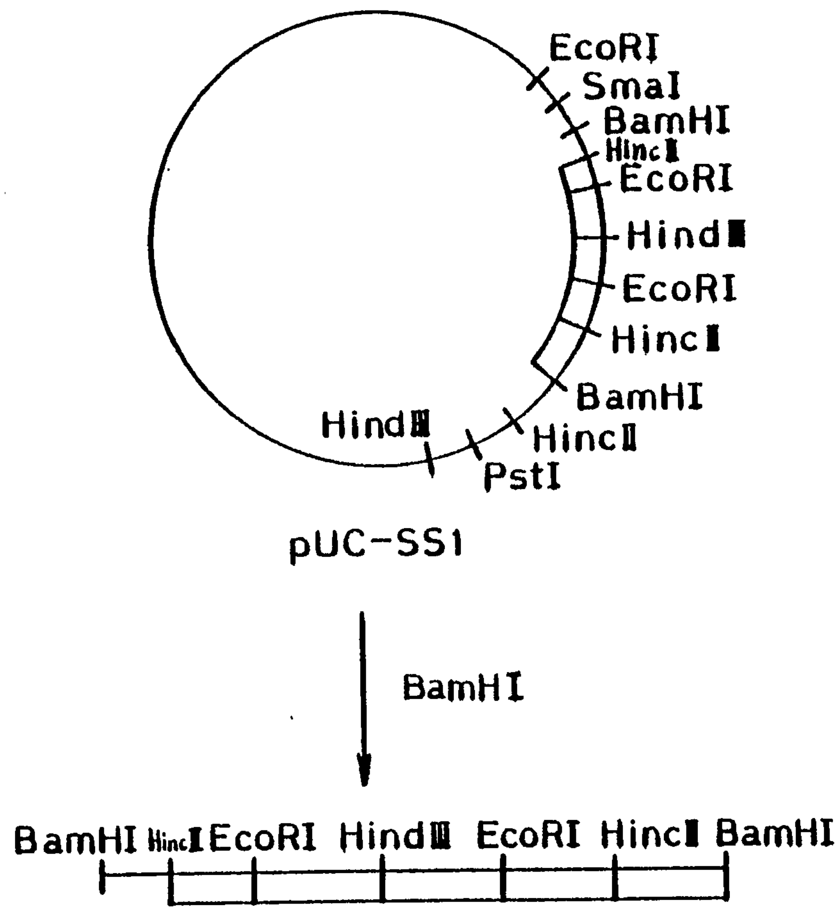


fused IGF-1 gene (464 bp)

Fig.12(a) Construction of plasmid pLS-T2 and pLS-T3 (part2)

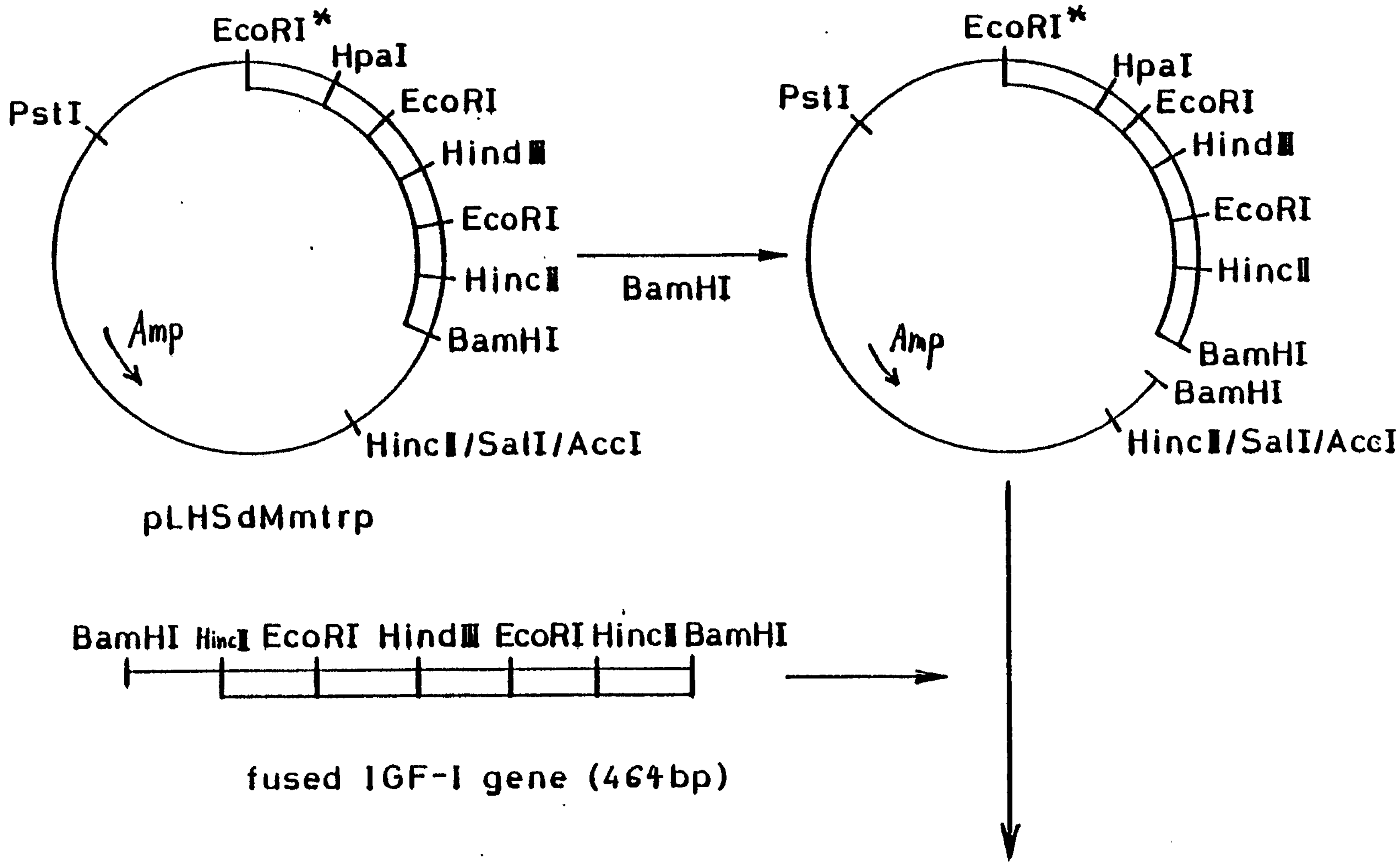

Fig.12(b) Construction of plasmid pLS-T2 and pLS-T3 (part2)

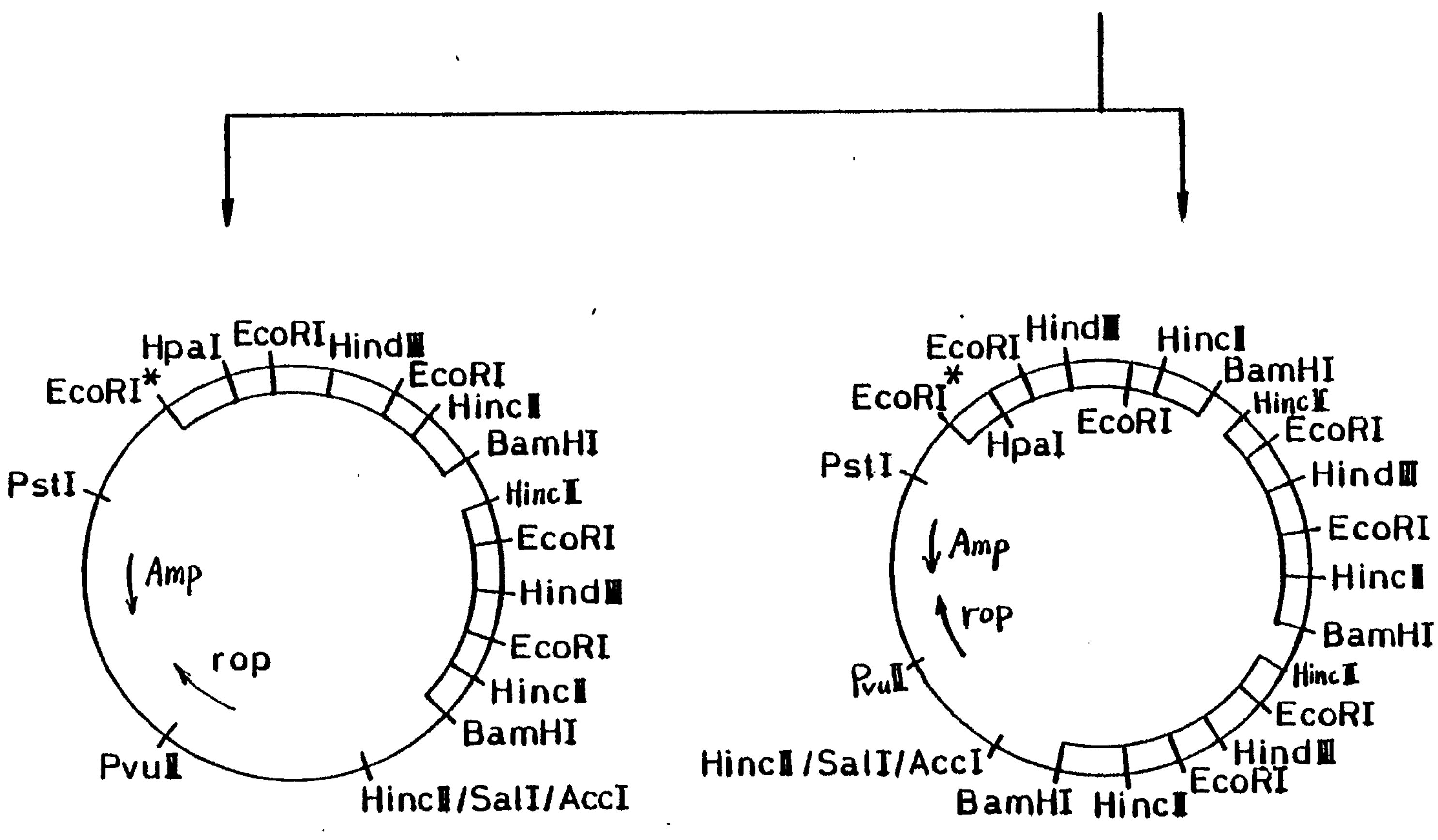


pLS-T2

pLS-T3

Fig.13 Construction of terminator S gene (47bp)

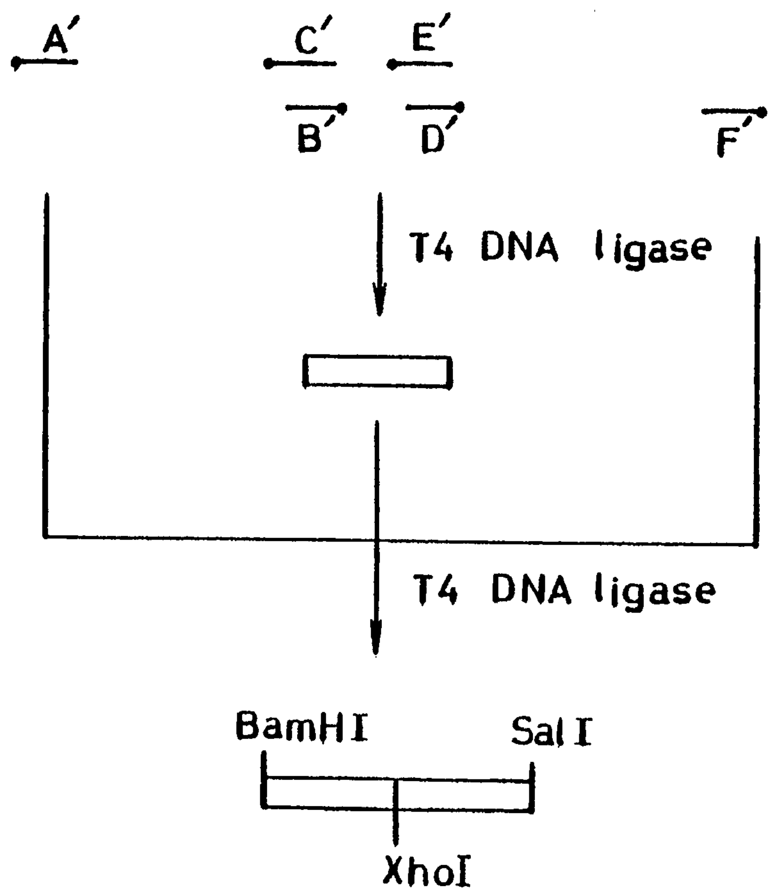


terminater S gene (47bp)

Fig.14 Construction of plasmid pTerS21

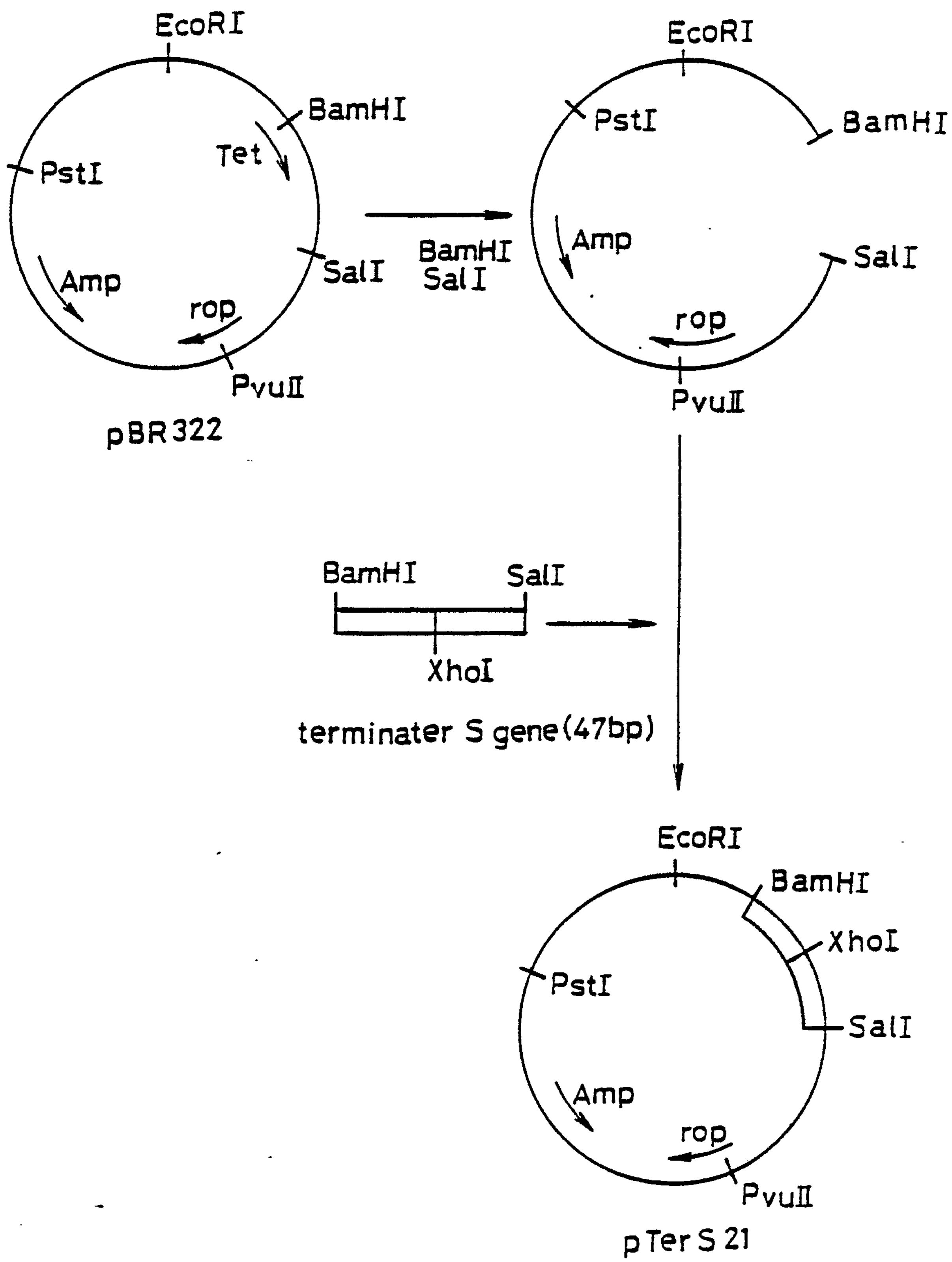

Fig.15 Construction of plasmid pLHSdMmTer

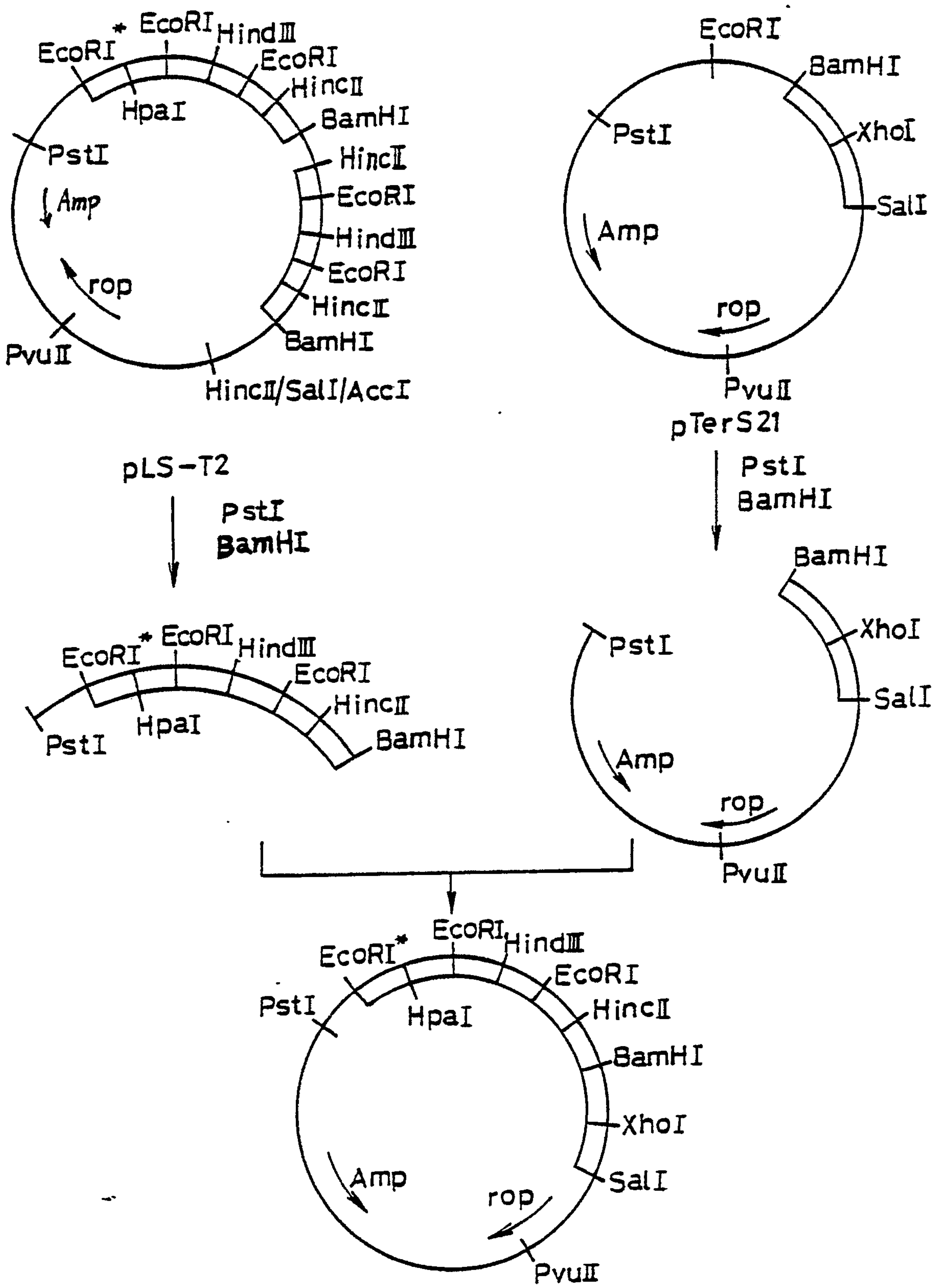


pLHSdMmTer

Fig.16 Construction of plasmid pLS−D1

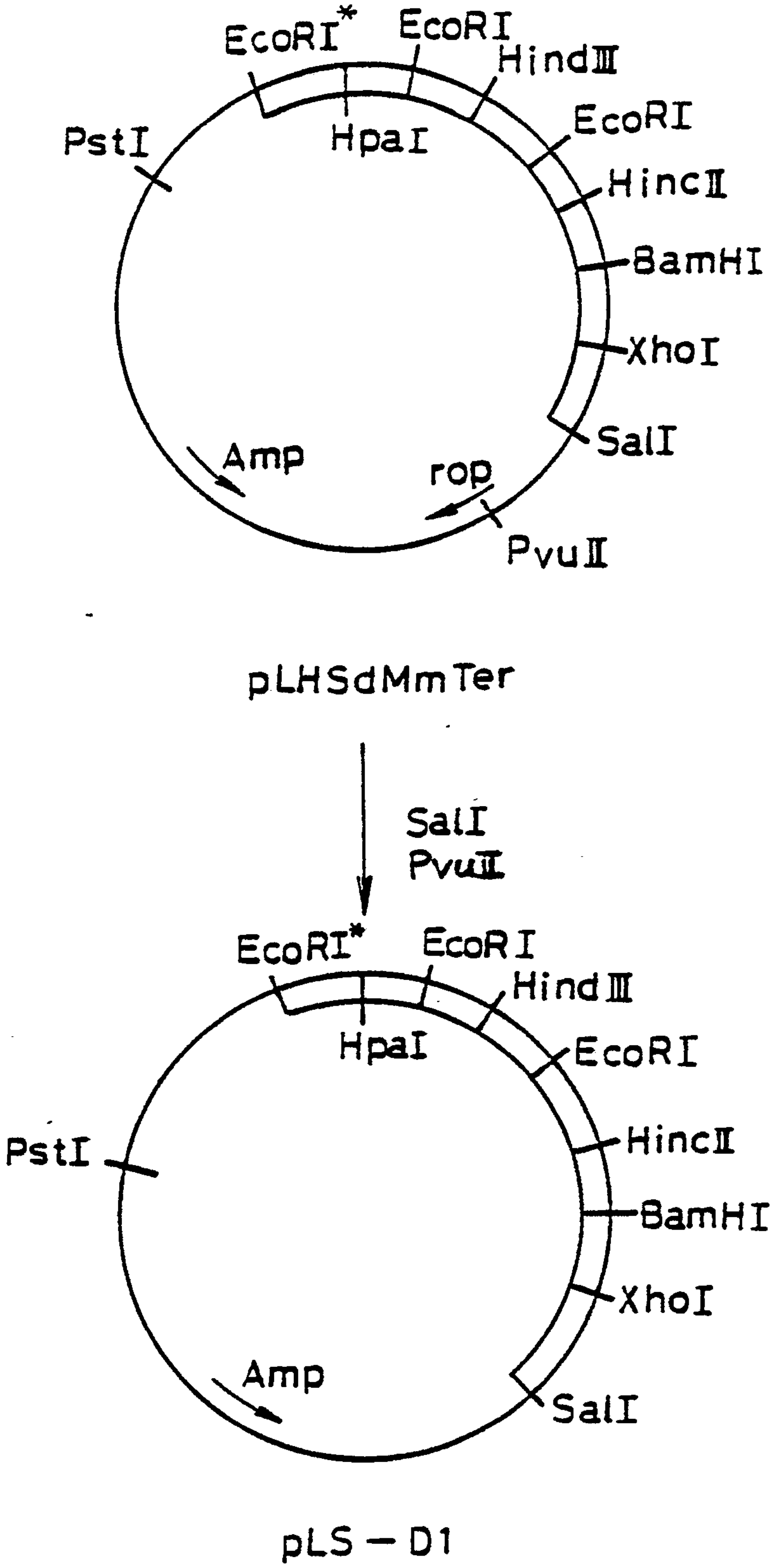

Fig.17 Construction of plasmid pLS-D2

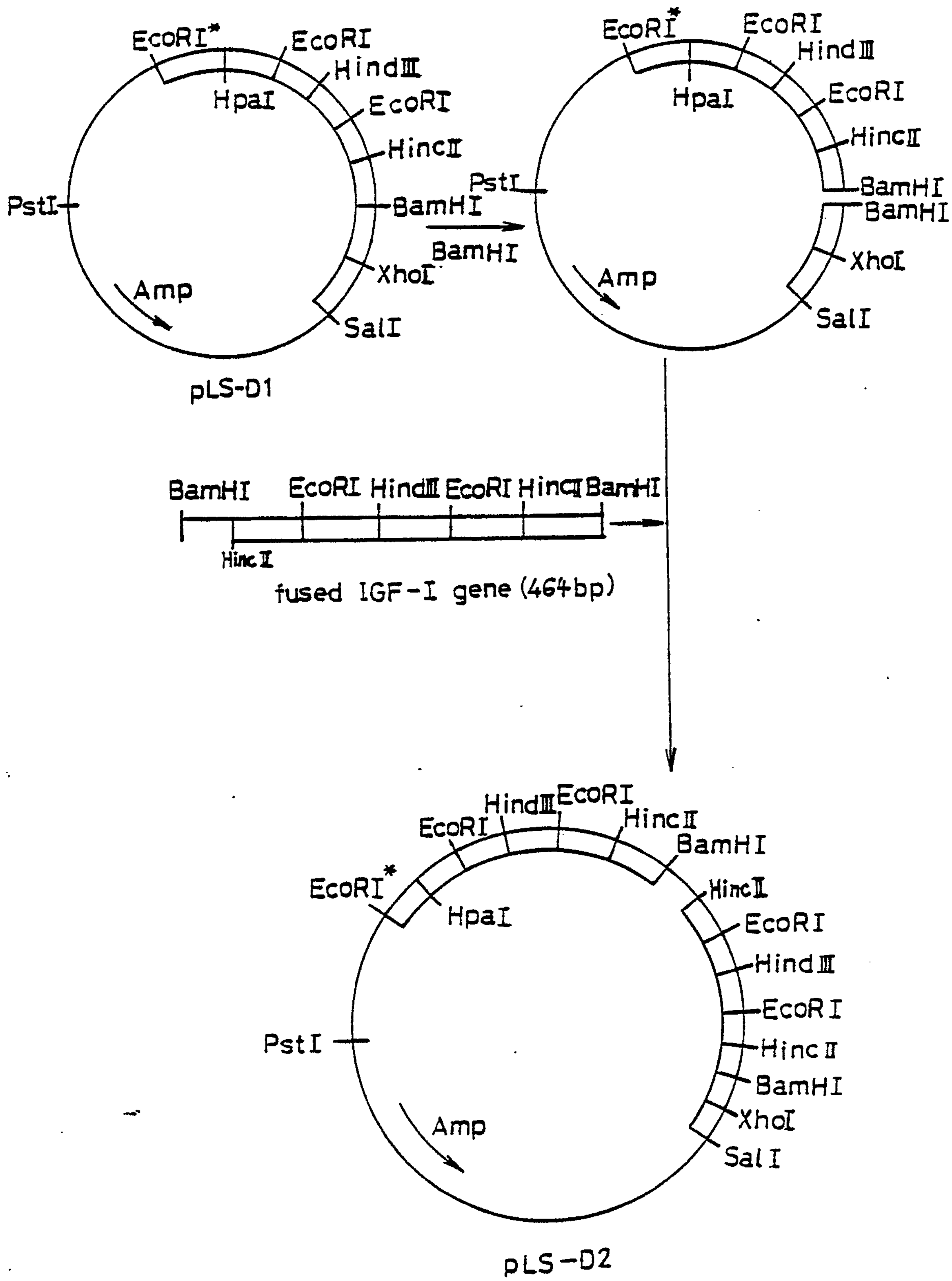

Fig.18(a) Construction of pLS-DD1

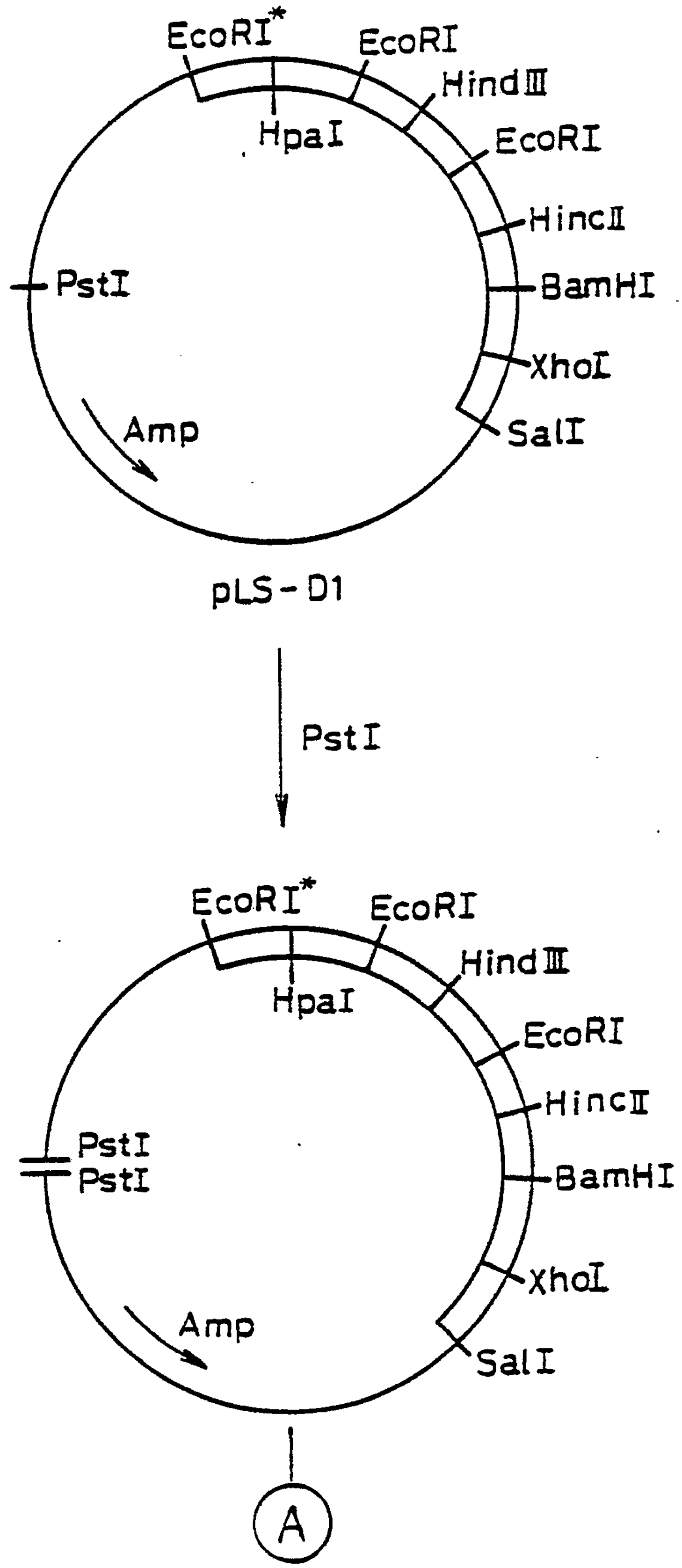

Fig.18(b) Construction of pLS-DD1

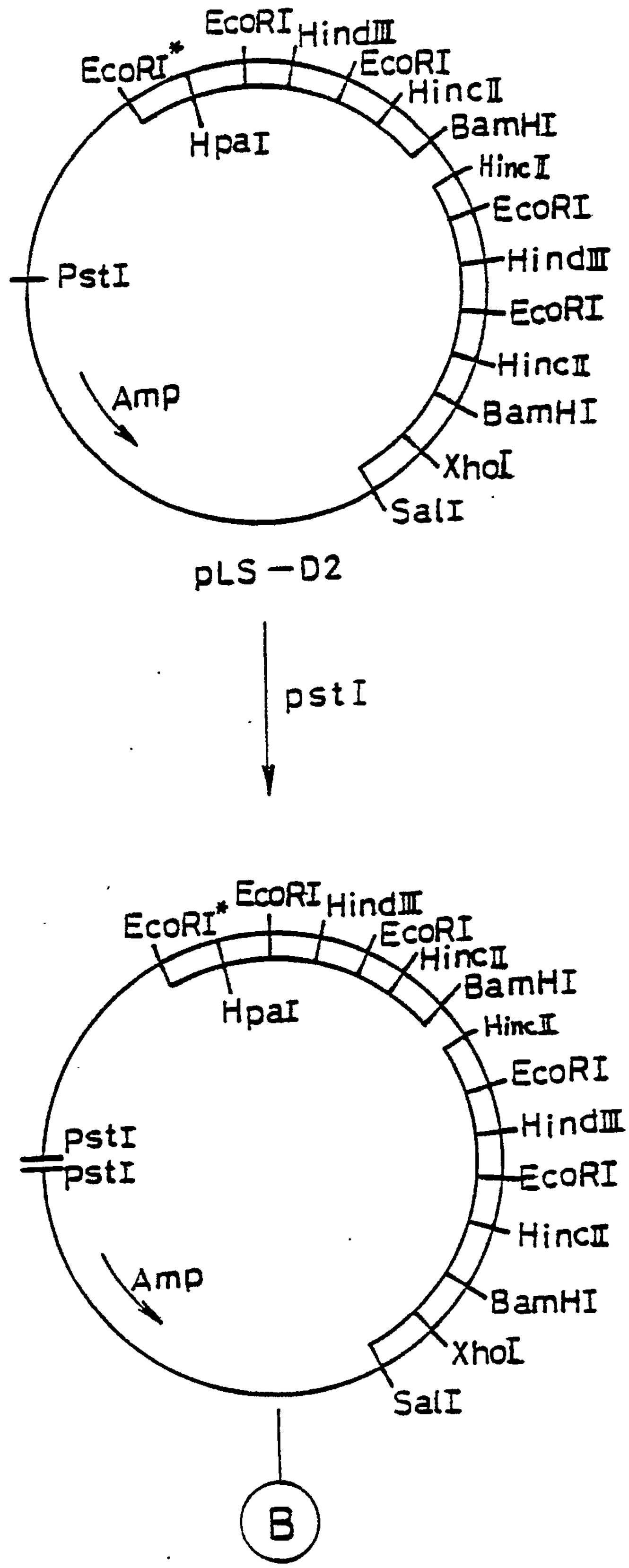

Fig.18(C) Construction of pLS DD1

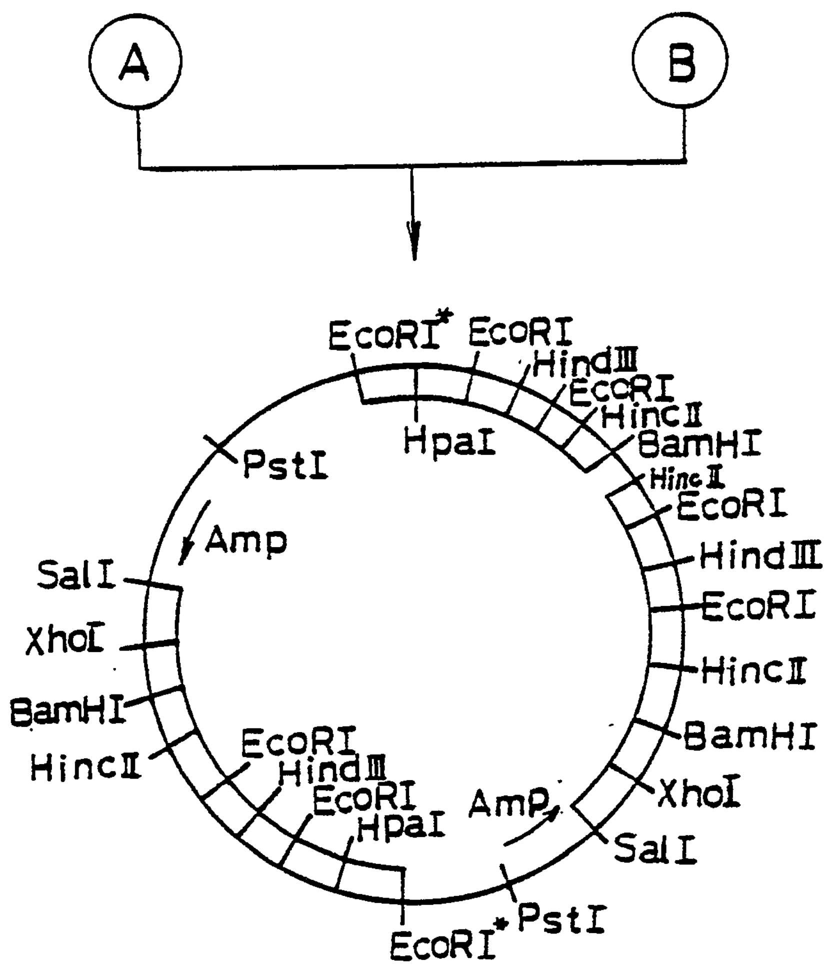


pLS—DD1

Fig. 19 Amino acid analysis and sequence analysis of IGF-I

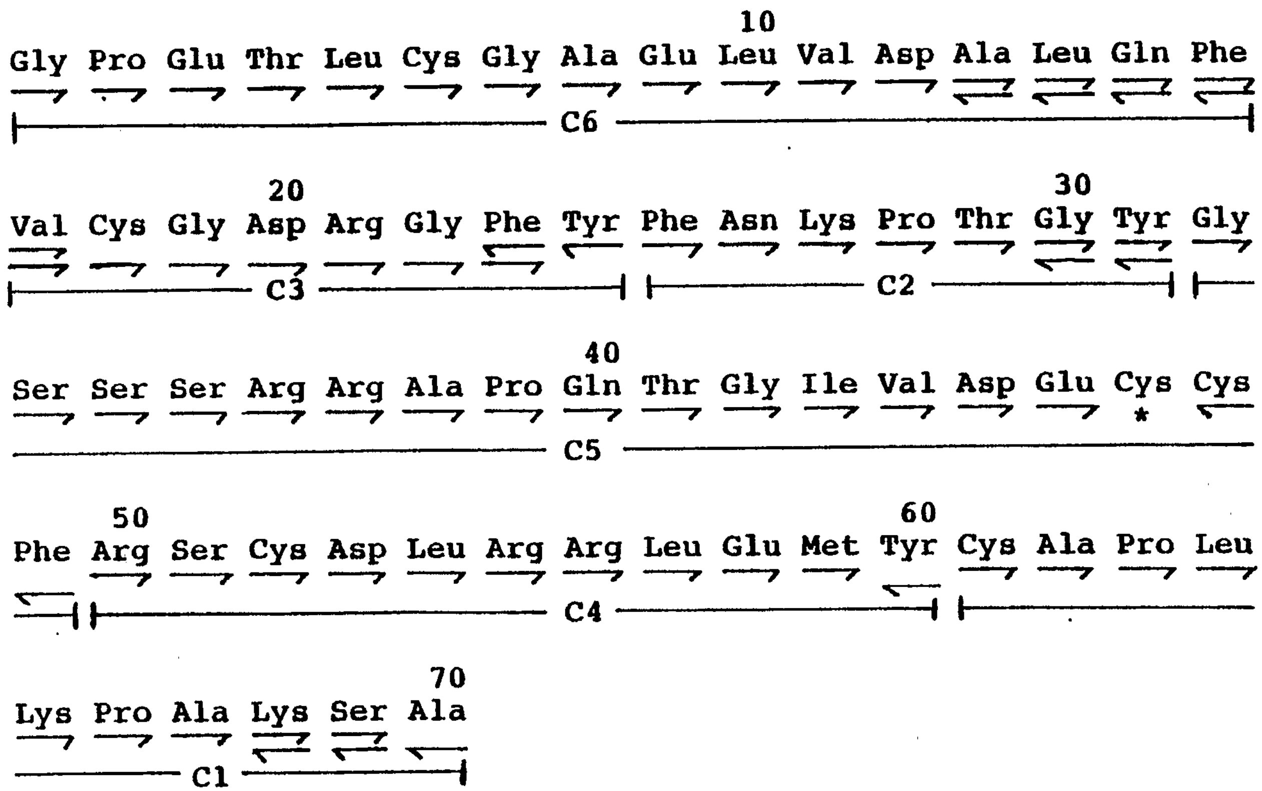


→ Amino acid sequence determined by Edman's method

← Amino acid sequence determined by carboxypeptidase method

C1 to C6 show the fragments digested with chymotrypsin

* determined by amino acid analysis